# EUROPEAN PATENT APPLICATION

(11) **EP 4 810 511 A1**
(43) Date of publication of application: **23.09.2026**
(21) Application number: 24891501.9
(22) Date of filing: 15.11.2024
(51) Int. Cl.: C07K 14/735, C07K 1/22, C07K 17/00, C12N 1/21, C12N 15/63, C12P 21/00

(54) **MODIFIED FC-BINDING PROTEIN AND METHOD FOR PRODUCING SAME**

(30) Priority: 17.11.2023 JP 2023195783; 05.03.2024 JP 2024033082; 18.10.2024 JP 2024184285
(71) Applicant: Tosoh Corporation, Shunan-shi, Yamaguchi 746-8501 (JP)
(72) Inventor: INOUE, Naruaki, Ayase-shi, Kanagawa 252-1123 (JP); WATANABE, Ryoko, Ayase-shi, Kanagawa 252-1123 (JP); YUMOTO, Tatsuya, Ayase-shi, Kanagawa 252-1123 (JP); HIRONO, Linko, Ayase-shi, Kanagawa 252-1123 (JP); TERAO, Yosuke, Ayase-shi, Kanagawa 252-1123 (JP)
(74) Representative: Novagraaf International SA
(86) International application number: PCT/JP2024/040645
(87) International publication number: WO 2025/105466

(57) **Abstract**

A problem to be addressed by the present invention is to provide a polypeptide that exhibits binding ability to a substance having an Fc region, such as an immunoglobulin, and has excellent productivity, and a method of producing the polypeptide. This problem is solved by an Fc-binding protein in which an amino acid at a specific position among the amino acids constituting the Fc-binding protein is substituted with another amino acid.

## Description

### Technical Field

The present disclosure relates to an Fc-binding protein having binding affinity for an Fc region possessed by an immunoglobulin (such as IgG) or the like. More specifically, the present disclosure relates to a human neonatal Fc receptor (human FcRn) with enhanced productivity, and a method of producing the receptor.

### Background Art

An Fc receptor is a receptor protein that binds to an Fc region of an immunoglobulin molecule, and binds to an immune complex of an antigen and an immunoglobulin to transmit a signal into a cell (Non-Patent Literature 1). Individual molecules recognize a single immunoglobulin isotype or the same group of immunoglobulin isotypes by a recognition domain on the Fc receptor, which belongs to the immunoglobulin superfamily. This determines which accessory cell is mobilized in an immune response.

Fc receptors can be further classified into several subtypes, including Fcγ receptors, which are receptors for immunoglobulin G (IgG), Fcα receptors, Fcε receptors, and the like. Each receptor is classified more finely. The Fcγ receptors can be classified into subtypes: FcγRI (CD64), FcγRIIa (CD32a), FcγRIIb (CD32b), FcγRIIc (CD32c), FcγRIIIa (CD16a), and FcγRIIIb (CD16b) (Non-Patent Literature 1 and 2).

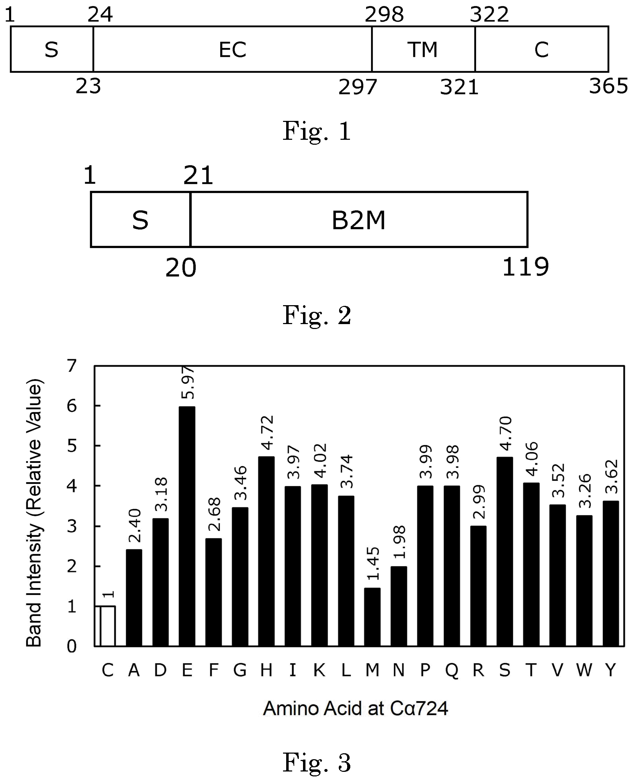

On the other hand, human FcRn is a major histocompatibility complex (MHC) class I-related molecule, and is composed of a heavy chain (α-chain) and β2 microglobulin (β-chain) (Non-Patent Literature 3). The amino acid sequence (SEQ ID NO: 1) of the α-chain of human FcRn is published in a public database such as UniProt (Accession number: P55899). Additionally, the amino acid sequence (SEQ ID NO: 2) of the β-chain is published in UniProt (Accession number: P61769). FIG. 1 shows a schematic structural diagram of the α-chain of human FcRn, and FIG. 2 shows a schematic structural diagram of the β-chain. Note that the amino acid numbers in FIG. 1 correspond to the amino acid numbers of SEQ ID NO: 1. That is, from methionine (M) at position 1 to glycine (G) at position 23 in SEQ ID NO: 1 is a signal sequence (S), from alanine (A) at position 24 to serine (S) at position 297 is an extracellular region (EC), from valine (V) at position 298 to tryptophan (W) at position 321 is a transmembrane region (TM), and from arginine (R) at position 322 to alanine (A) at position 365 is considered to be an intracellular region (C). Additionally, the amino acid numbers in FIG. 2 correspond to the amino acid numbers of SEQ ID NO: 2. That is, from methionine (M) at position 1 to alanine (A) at position 20 in SEQ ID NO: 2 is a signal sequence (S), and from isoleucine (I) at position 21 to methionine (M) at position 119 is considered to be β2 microglobulin (B2M).

The binding between human FcRn and IgG (Fc region) is pH-dependent, and binding occurs at pH 6.0 to 6.5, and dissociation occurs at pH 7.4 or higher. This pH dependence is involved in the IgG recycling mechanism and IgG transport mechanism of human FcRn in the body. It is known that IgG and Fc fusion proteins that bind to and dissociate from human FcRn in a pH-dependent manner have a long in vivo lifetime (Non-Patent Literature 4). A method of evaluating the in vivo lifetime of human IgG, utilizing this characteristic of human FcRn, and using an affinity column with recombinant human FcRn employed as a ligand is known (Non-Patent Literature 5).

As described above, recombinant human FcRn has the property of functioning as a ligand for an affinity column. Recombinant human FcRn with various improved functions and methods of producing the recombinant human FcRn have been disclosed hitherto (Patent Literature 1 to 4); however, further enhancement in productivity has been demanded.

### Citation List

### Patent Literature

Patent Literature 1: JP2018-183087A
Patent Literature 2: JP2021-073883A
Patent Literature 3: JP2021-136967A
Patent Literature 4: JP2022-076998A

### Non-Patent Literature

Non-Patent Literature 1: Takai T., Jpn. J. Clin. Immunol., 28, 318-326, 2005
Non-Patent Literature 2: J. Galon et al., Eur. J. Immunol., 27, 1928-1932, 1997
Non-Patent Literature 3: N. E. Simister et al., Nature, 337, 184-187, 1989
Non-Patent Literature 4: M. Raghavan et al., Biochemistry, 34, 14649-14657, 1995
Non-Patent Literature 5: F. Cymer et al., Bioanalysis, 9, 1305-1317, 2017

### Summary of Invention

### Technical Problem

A problem to be addressed by one aspect of the present disclosure is to provide: a polypeptide that exhibits binding ability to a substance having an Fc region, such as an immunoglobulin, and has excellent productivity; and a method of producing the polypeptide. Additionally, in one aspect, a problem is to provide: a polypeptide that exhibits binding ability to a substance having an Fc region, such as an immunoglobulin, and has excellent alkali resistance; and a method of producing the polypeptide.

### Solution to Problem

The present inventors have conducted intensive studies to solve the above problems and have consequently discovered that the productivity of the Fc-binding protein is enhanced by substituting an amino acid residue at a specific position among the amino acids constituting the Fc-binding protein with another specific amino acid residue.

That is, the present disclosure includes the aspects according to the following [1] to [11].
[1] An Fc-binding protein selected from any one of the following <a> to <c>:
   <a> an Fc-binding protein including amino acid residues from alanine at position 24 to serine at position 297 of the amino acid sequence of SEQ ID NO: 1 and amino acid residues from isoleucine at position 21 to methionine at position 119 of the amino acid sequence of SEQ ID NO: 2, with the proviso that at least the amino acid substitutions of the following (1) to (7) are introduced into the amino acid residues:
      (1) substitution of cysteine at position 71 of SEQ ID NO: 1 with arginine;
      (2) substitution of asparagine at position 78 of SEQ ID NO: 1 with aspartic acid;
      (3) substitution of arginine at position 192 of SEQ ID NO: 1 with leucine;
      (4) substitution of asparagine at position 196 of SEQ ID NO: 1 with aspartic acid;
      (5) substitution of glutamine at position 232 of SEQ ID NO: 1 with leucine;
      (6) substitution of lysine at position 295 of SEQ ID NO: 1 with glutamic acid; and
      (7) substitution of cysteine at position 274 of SEQ ID NO: 1 with another amino acid;
   <b> an Fc-binding protein including amino acid residues from alanine at position 24 to serine at position 297 of the amino acid sequence of SEQ ID NO: 1 and amino acid residues from isoleucine at position 21 to methionine at position 119 of the amino acid sequence of SEQ ID NO: 2, with the proviso that, into the amino acid residues, the amino acid substitutions of (1) to (7) above are introduced, and furthermore, any one or more of substitutions, deletions, insertions, and additions of one or several amino acid residues at one or several positions other than the amino acid substitutions of (1) to (7) above are introduced, and wherein the Fc-binding protein has antibody-binding activity; and
   <c> an Fc-binding protein including an amino acid sequence that has an identity of 70% or more to the entire amino acid sequence composed of the amino acid sequence from alanine at position 24 to serine at position 297 of the amino acid sequence of SEQ ID NO: 1 and the amino acid sequence from isoleucine at position 21 to methionine at position 119 of the amino acid sequence of SEQ ID NO: 2, with the proviso that the amino acid substitutions of (1) to (7) above are introduced into the entire amino acid sequence, wherein the amino acid sequence of the Fc-binding protein has the amino acid substitutions of (1) to (7) above maintained therein, and wherein the Fc-binding protein has antibody-binding activity.
[2] The Fc-binding protein according to [1], selected from any one of the following <d> to <f>:
   <d> an Fc-binding protein including amino acid residues from alanine at position 24 to serine at position 297 of the amino acid sequence of SEQ ID NO: 1 and amino acid residues from isoleucine at position 21 to methionine at position 119 of the amino acid sequence of SEQ ID NO: 2, with the proviso that the amino acid substitutions of (1) to (7) above and any one or more of the amino acid substitutions of the following (8) to (10) are introduced into the amino acid residues:
      (8) substitution of valine at position 80 of SEQ ID NO: 1 with aspartic acid;
      (9) substitution of lysine at position 96 of SEQ ID NO: 1 with glutamic acid; and
      (10) substitution of asparagine at position 172 of SEQ ID NO: 1 with aspartic acid;
   <e> an Fc-binding protein including amino acid residues from alanine at position 24 to serine at position 297 of the amino acid sequence of SEQ ID NO: 1 and amino acid residues from isoleucine at position 21 to methionine at position 119 of the amino acid sequence of SEQ ID NO: 2, with the proviso that, into the amino acid residues, the amino acid substitutions of (1) to (7) above and at least any one of the amino acid substitutions of (8) to (10) above are introduced, and furthermore, any one or more of substitutions, deletions, insertions, and additions of one or several amino acid residues at one or several positions other than the amino acid substitutions of (1) to (10) above are introduced, and wherein the Fc-binding protein has antibody-binding activity; and
   <f> an Fc-binding protein including an amino acid sequence that has an identity of 70% or more to the entire amino acid sequence composed of the amino acid sequence from alanine at position 24 to serine at position 297 of the amino acid sequence of SEQ ID NO: 1 and the amino acid sequence from isoleucine at position 21 to methionine at position 119 of the amino acid sequence of SEQ ID NO: 2, with the proviso that the amino acid substitutions of (1) to (7) above and at least any one of the amino acid substitutions of (8) to (10) above are introduced into the entire amino acid sequence, wherein the amino acid sequence of the Fc-binding protein has the amino acid substitutions of (1) to (10) above maintained therein, and wherein the Fc-binding protein has antibody-binding activity.
[3] The Fc-binding protein according to [1] or [2], selected from any one of the following <g> to <i>:
   <g> an Fc-binding protein including amino acid residues from alanine at position 24 to serine at position 297 of the amino acid sequence of SEQ ID NO: 1 and amino acid residues from isoleucine at position 21 to methionine at position 119 of the amino acid sequence of SEQ ID NO: 2, with the proviso that at least the amino acid substitutions of (1) to (10) above are introduced into the amino acid residues;
   <h> an Fc-binding protein including amino acid residues from alanine at position 24 to serine at position 297 of the amino acid sequence of SEQ ID NO: 1 and amino acid residues from isoleucine at position 21 to methionine at position 119 of the amino acid sequence of SEQ ID NO: 2, with the proviso that, into the amino acid residues, the amino acid substitutions of (1) to (10) above are introduced, and furthermore, any one or more of substitutions, deletions, insertions, and additions of one or several amino acid residues at one or several positions other than the amino acid substitutions of (1) to (10) above are introduced, and wherein the Fc-binding protein has antibody-binding activity; and
   <i> an Fc-binding protein including an amino acid sequence that has an identity of 70% or more to the entire amino acid sequence composed of the amino acid sequence from alanine at position 24 to serine at position 297 of the amino acid sequence of SEQ ID NO: 1 and the amino acid sequence from isoleucine at position 21 to methionine at position 119 of the amino acid sequence of SEQ ID NO: 2, with the proviso that the amino acid substitutions of (1) to (10) above are introduced into the entire amino acid sequence, wherein the amino acid sequence of the Fc-binding protein has the amino acid substitutions of (1) to (10) above maintained therein, and wherein the Fc-binding protein has antibody-binding activity.
[4] The Fc-binding protein according to any one of [1] to [3], wherein the other amino acid in (7) above is any one of serine, glutamic acid, and histidine.
[5] A polynucleotide encoding the Fc-binding protein according to any one of [1] to [4].
[6] An expression vector including the polynucleotide according to [5].
[7] A transformant capable of producing an Fc-binding protein, obtained by transforming a host with the expression vector according to [6].
[8] The transformant according to [7], wherein the host is *Escherichia coli*.
[9] A method of producing an Fc-binding protein, including the steps of: culturing the transformant according to [7] or [8] to produce the Fc-binding protein; and collecting the produced Fc-binding protein from the resultant culture product.
[10] An antibody adsorbent obtained by immobilizing the Fc-binding protein according to any one of [1] to [4] on an insoluble carrier.
[11] A method of separating a substance having an Fc region, including the steps of: adding a solution containing a substance having an Fc region to a column packed with the adsorbent according to [10] to adsorb the substance having an Fc region to the adsorbent; and eluting the substance having an Fc region adsorbed to the adsorbent, using an eluent.

### Brief Description of Drawings

FIG. 1 is a schematic diagram of the α-chain of human FcRn. The numerals in the figure indicate the numbers in the amino acid sequence of SEQ ID NO: 1. In the figure, S indicates a signal sequence, EC indicates an extracellular region, TM indicates a transmembrane region, and C indicates an intracellular region.
FIG. 2 is a schematic diagram of the β-chain of human FcRn. The numerals in the figure indicate the numbers in the amino acid sequence of SEQ ID NO: 2. In the figure, S indicates a signal sequence, and B2M indicates β2 microglobulin.
FIG. 3 is a diagram showing the results of comparing the expression level (band intensity) of Fc-binding proteins in which cysteine at position 274 of SEQ ID NO: 1 (Cα274) is substituted with another amino acid. Note that the band intensity is expressed as a relative value, with the value in the unsubstituted state (that is, Cα274 as is) set to 1.
FIG. 4 is a diagram showing the results of comparing purification yields between FcRnm7b-2 (abbreviated as m7b in the figure, corresponding to isoleucine (I) at position 22 to glycine (G) at position 432 of SEQ ID NO: 92) and FcRnm10 (abbreviated as m10 in the figure, corresponding to isoleucine (I) at position 22 to glycine (G) at position 432 of SEQ ID NO: 93). Note that the purification yield is expressed as a relative value, with the purification yield of m7b set to 1.
FIG. 5 is a diagram showing the results of measuring the binding activity of FcRnm10 (corresponding to isoleucine (I) at position 22 to glycine (G) at position 432 of SEQ ID NO: 93) to IgG.
FIG. 6 is a diagram showing the results of separating a monoclonal antibody (Rituxan, Zenyaku Kogyo Co., Ltd.) using a column packed with a gel on which FcRnm10 (corresponding to isoleucine (I) at position 22 to glycine (G) at position 432 of SEQ ID NO: 93) is immobilized.
FIG. 7 is a diagram showing the results of separating various antibodies using a column packed with a gel on which FcRnm10 (corresponding to isoleucine (I) at position 22 to glycine (G) at position 432 of SEQ ID NO: 93) is immobilized. <No.> in the figure corresponds to the antibodies listed in Table 6.
FIG. 8 is a diagram showing the results of alkali resistance evaluation of Fc-binding proteins. WT indicates FcRnWT, m7a indicates FcRnm7a, m7b indicates FcRnm7b, and m10 indicates FcRnm10.

### Description of Embodiments

Hereinafter, the present disclosure will be described in detail. In the present specification and the appended claims, the singular forms "a", "an", and "the" include plural referents unless the context clearly dictates otherwise.

An Fc-binding protein of the present disclosure is a protein having binding ability to an Fc region, including at least amino acid residues of the following (I) and (II), with the proviso that the protein has amino acid substitutions at specific positions in the amino acid residues:
(I) amino acid residues from alanine at position 24 to serine at position 297, which correspond to the extracellular region (region EC in FIG. 1) of the human FcRn α-chain having the amino acid sequence of SEQ ID NO: 1;
(II) amino acid residues from isoleucine at position 21 to methionine at position 119, which correspond to the β2 microglobulin region (region B2M in FIG. 2) of the human FcRn β-chain having the amino acid sequence of SEQ ID NO: 2.

Accordingly, the Fc-binding protein of the present disclosure may include all or part of the signal peptide region (region S in FIG. 1 and FIG. 2) located on the N-terminal side of each of the extracellular region (region EC in FIG. 1) of the human FcRn α-chain and the β2 microglobulin region (region B2M in FIG. 2) of the human FcRn β-chain. Additionally, the Fc-binding protein may include all or part of the transmembrane region (region TM in FIG. 1) and the extracellular region (region C in FIG. 1) located on the C-terminal side of the extracellular region (region EC in FIG. 1) of the human FcRn α-chain.

In the present specification, the Fc region refers to an Fc region of an immunoglobulin. Immunoglobulin may be read as antibody. That is, the Fc region of an immunoglobulin is also referred to as an Fc region of an antibody. A protein having binding ability to an Fc region may have binding ability not only to an antibody but also to any substance among substances having an Fc region. In other words, the substance having an Fc region is a substance having an Fc region of an antibody. And the substance having an Fc region may be a substance composed of an Fc region of an antibody and an arbitrary substance, such as, a protein having an arbitrary amino acid sequence or an arbitrary small molecule compound. Examples of the substance having an Fc region include antibodies, Fc fusion proteins, and complexes of an Fc region and a drug. An Fc-binding protein of the present disclosure, which is a protein having binding ability to an Fc region, may be a protein capable of binding to any substance having an Fc region, including the substances exemplified above.

In the present specification, the Fc-binding protein including at least the amino acid residues of (I) above and (II) above includes at least the amino acid sequence of (I) above and the amino acid sequence of (II) above in the amino acid sequence of the protein. The order of the amino acid residues of (I) above and the amino acid residues of (II) above does not matter. That is, the amino acid residues of (II) above may be located on the N-terminal side or on the C-terminal side of the amino acid residues of (I) above. Additionally, the amino acid residues of (I) above and the amino acid residues of (II) above may be directly linked in one aspect or may be linked via a known linker such as a GS linker (for example, a linker composed of a repetition of four glycine (G) residues and one serine (S) residue), in another aspect. The length of the linker may be arbitrary and is not particularly limited. Specifically, the length of the linker may be, for example, 5 to 50 amino acid residues.

Specifically, the amino acid substitutions at specific positions refer to amino acid substitutions of the following (1) to (7). It has been found that the productivity of the Fc-binding protein is enhanced by having the amino acid substitutions described below.
(1) substitution of cysteine at position 71 of SEQ ID NO: 1 with arginine;
(2) substitution of asparagine at position 78 of SEQ ID NO: 1 with aspartic acid;
(3) substitution of arginine at position 192 of SEQ ID NO: 1 with leucine;
(4) substitution of asparagine at position 196 of SEQ ID NO: 1 with aspartic acid;
(5) substitution of glutamine at position 232 of SEQ ID NO: 1 with leucine;
(6) substitution of lysine at position 295 of SEQ ID NO: 1 with glutamic acid; and
(7) substitution of cysteine at position 274 of SEQ ID NO: 1 with another amino acid.

In one aspect, the Fc-binding protein may be a protein described below.
<a> An Fc-binding protein including amino acid residues from alanine at position 24 to serine at position 297 of the amino acid sequence of SEQ ID NO: 1 and amino acid residues from isoleucine at position 21 to methionine at position 119 of the amino acid sequence of SEQ ID NO: 2, with the proviso that at least the amino acid substitutions of the following (1) to (7) are introduced into the amino acid residues:
   (1) substitution of cysteine at position 71 of SEQ ID NO: 1 with arginine;
   (2) substitution of asparagine at position 78 of SEQ ID NO: 1 with aspartic acid;
   (3) substitution of arginine at position 192 of SEQ ID NO: 1 with leucine;
   (4) substitution of asparagine at position 196 of SEQ ID NO: 1 with aspartic acid;
   (5) substitution of glutamine at position 232 of SEQ ID NO: 1 with leucine;
   (6) substitution of lysine at position 295 of SEQ ID NO: 1 with glutamic acid; and
   (7) substitution of cysteine at position 274 of SEQ ID NO: 1 with another amino acid.
<b> An Fc-binding protein including amino acid residues from alanine at position 24 to serine at position 297 of the amino acid sequence of SEQ ID NO: 1 and amino acid residues from isoleucine at position 21 to methionine at position 119 of the amino acid sequence of SEQ ID NO: 2, with the proviso that, into the amino acid residues, the amino acid substitutions of (1) to (7) above are introduced, and furthermore, any one or more of substitutions, deletions, insertions, and additions of one or several amino acid residues at one or several positions other than the amino acid substitutions of (1) to (7) above are introduced, and wherein the Fc-binding protein has antibody-binding activity.
<c> An Fc-binding protein including an amino acid sequence that has an identity of 70% or more to the entire amino acid sequence composed of the amino acid sequence from alanine at position 24 to serine at position 297 of the amino acid sequence of SEQ ID NO: 1 and the amino acid sequence from isoleucine at position 21 to methionine at position 119 of the amino acid sequence of SEQ ID NO: 2, with the proviso that the amino acid substitutions of (1) to (7) above are introduced into the entire amino acid sequence, wherein the amino acid sequence of the Fc-binding protein has the amino acid substitutions of (1) to (7) above maintained therein, and wherein the Fc-binding protein has antibody-binding activity.
<d> An Fc-binding protein including amino acid residues from alanine at position 24 to serine at position 297 of the amino acid sequence of SEQ ID NO: 1 and amino acid residues from isoleucine at position 21 to methionine at position 119 of the amino acid sequence of SEQ ID NO: 2, with the proviso that the amino acid substitutions of (1) to (7) above and at least any one or more of the amino acid substitutions of the following (8) to (10) are introduced into the amino acid residues:
   (8) substitution of valine at position 80 of SEQ ID NO: 1 with aspartic acid;
   (9) substitution of lysine at position 96 of SEQ ID NO: 1 with glutamic acid; and
   (10) substitution of asparagine at position 172 of SEQ ID NO: 1 with aspartic acid.
<e> An Fc-binding protein including amino acid residues from alanine at position 24 to serine at position 297 of the amino acid sequence of SEQ ID NO: 1 and amino acid residues from isoleucine at position 21 to methionine at position 119 of the amino acid sequence of SEQ ID NO: 2, with the proviso that, into the amino acid residues, the amino acid substitutions of (1) to (7) above and at least any one of the amino acid substitutions of (8) to (10) above are introduced, and furthermore, any one or more of substitutions, deletions, insertions, and additions of one or several amino acid residues at one or several positions other than the amino acid substitutions of (1) to (10) above are introduced, and wherein the Fc-binding protein has antibody-binding activity.
<f> An Fc-binding protein including an amino acid sequence that has an identity of 70% or more to the entire amino acid sequence composed of the amino acid sequence from alanine at position 24 to serine at position 297 of the amino acid sequence of SEQ ID NO: 1 and the amino acid sequence from isoleucine at position 21 to methionine at position 119 of the amino acid sequence of SEQ ID NO: 2, with the proviso that the amino acid substitutions of (1) to (7) above and at least any one of the amino acid substitutions of (8) to (10) above are introduced into the entire amino acid sequence, wherein the amino acid sequence of the Fc-binding protein has the amino acid substitutions of (1) to (10) above maintained therein, and wherein the Fc-binding protein has antibody-binding activity.
<g> An Fc-binding protein including amino acid residues from alanine at position 24 to serine at position 297 of the amino acid sequence of SEQ ID NO: 1 and amino acid residues from isoleucine at position 21 to methionine at position 119 of the amino acid sequence of SEQ ID NO: 2, with the proviso that at least the amino acid substitutions of (1) to (10) above are introduced into the amino acid residues.
<h> An Fc-binding protein including amino acid residues from alanine at position 24 to serine at position 297 of the amino acid sequence of SEQ ID NO: 1 and amino acid residues from isoleucine at position 21 to methionine at position 119 of the amino acid sequence of SEQ ID NO: 2, with the proviso that, into the amino acid residues, at least the amino acid substitutions of (1) to (10) above are introduced, and furthermore, any one or more of substitutions, deletions, insertions, and additions of one or several amino acid residues at one or several positions other than the amino acid substitutions of (1) to (10) above are introduced, and wherein the Fc-binding protein has antibody-binding activity.
<i> An Fc-binding protein including an amino acid sequence that has an identity of 70% or more to the entire amino acid sequence composed of the amino acid sequence from alanine at position 24 to serine at position 297 of the amino acid sequence of SEQ ID NO: 1 and the amino acid sequence from isoleucine at position 21 to methionine at position 119 of the amino acid sequence of SEQ ID NO: 2, with the proviso that the amino acid substitutions of (1) to (10) above are introduced into the entire amino acid sequence, wherein the amino acid sequence of the Fc-binding protein has the amino acid substitutions of (1) to (10) above maintained therein, and wherein the Fc-binding protein has antibody-binding activity.

An Fc-binding protein of the present disclosure may have at least the amino acid substitutions at the specific positions described above. As long as the Fc-binding protein has antibody-binding activity, the Fc-binding protein may further include any one or more of substitutions, deletions, insertions, and additions (hereinafter also collectively referred to as "modifications") of amino acid residues other than the amino acid substitutions at the specific positions described above.

In <b>, <e>, and <h> above, "one or several" means, for example, any of 1 to 50, 1 to 40, 1 to 35, 1 to 30, 1 to 25, 1 to 20, 1 to 15, 1 to 10, 1 to 9, 1 to 8, 1 to 7, 1 to 6, 1 to 5, 1 to 4, 1 to 3, and 1 to 2, although "one or several" varies depending on the position of the amino acid residue or the type of the amino acid residue in the three-dimensional structure of the protein. Note that the position of modification of the amino acid residue may be any position in the amino acid residue portion from alanine at position 24 to serine at position 297 of the amino acid sequence of SEQ ID NO: 1 and the amino acid residue portion from isoleucine at position 21 to methionine at position 119 of the amino acid sequence of SEQ ID NO: 2, other than the positions explicitly excluded in <b>, <e>, and <h> above. Additionally, the modifications of "one or several" amino acid residues may be introduced at a position other than the positions of modification disclosed in, for example, JP2018-183087A, JP2021-073883A, JP2021-136967A, and JP2022-076998A, as long as the Fc-binding protein has antibody-binding activity. Additionally, "any one or more of substitutions, deletions, insertions, and additions" may include a mutant or variant that can arise naturally, based on, for example, individual differences or species differences of a microorganism from which a gene is derived.

The identity of the amino acid sequence in <c>, <f>, and <i> above may be 70% or more, and the identity may be higher than that, for example, 80% or more, 85% or more, 90% or more, or 95% or more.

In the present specification, the "identity" of amino acid sequences refers to a value expressed as a percentage, calculated by aligning two amino acid sequences to be compared so that as many amino acid residues as possible can match between the amino acid sequences, and dividing the number of matched amino acid residues by the total number of amino acid residues. In the alignment, a gap is inserted as appropriate into one or both of the two sequences to be compared, if necessary. A method for such sequence alignment is not particularly limited, but can be performed, for example, using a well-known sequence comparison program such as BLAST, FASTA, or CLUSTALW. In a case where a gap is inserted, the total number of amino acid residues is the number of residues calculated by counting one gap as one amino acid residue. When the total number of amino acid residues thus counted differs between the two sequences to be compared, the sequence identity (%) is calculated as the number of matched amino acid residues divided by the total number of amino acid residues of the longer sequence.

In the present specification, "antibody-binding activity" means an activity of binding to an antibody as a target molecule. "Antibody-binding activity" in <b>, <e>, <h>, <c>, <f>, and <i> above may mean an activity of binding to an Fc region, that is, Fc-binding activity.

An Fc-binding protein of the present disclosure may further have a conservative substitution, which means a substitution is made between amino acids having similar physical properties and/or chemical properties. It is known to those skilled in the art that, without limitation to the Fc-binding protein, a conservative substitution generally allows a protein having a substitution introduced thereinto to maintain the function of a protein having no substitution introduced thereinto. Examples of the conservative substitution include substitutions between glycine and alanine, between aspartic acid and glutamic acid, between serine and proline, or between glutamic acid and alanine (Protein Structure and Function, Medical Science International, Ltd., 9, 2005).

Additionally, cysteine at position 274 of SEQ ID NO: 1 is preferably substituted with any one of glutamic acid, histidine, and serine in terms of further enhancing productivity.

The Fc-binding protein may further have an oligopeptide added to the N-terminal side or the C-terminal side thereof, in which the oligopeptide is useful when the Fc-binding protein is separated from a solution in the presence of contaminants. Examples of the oligopeptide include polyhistidine, polylysine, polyarginine, polyglutamic acid, and polyaspartic acid. Furthermore, an oligopeptide containing cysteine, which oligopeptide is useful for immobilizing the Fc-binding protein on a solid phase such as a support for chromatography, may be further added to the N-terminal side or the C-terminal side of the Fc-binding protein. The length of the oligopeptide added to the N-terminal side or the C-terminal side of the Fc-binding protein is not particularly limited as long as the antibody-binding ability and stability of the Fc-binding protein are not impaired. When the oligopeptide is added to the Fc-binding protein, a polynucleotide encoding the oligopeptide may be created and then added to the N-terminal side or the C-terminal side of the Fc-binding protein by genetic engineering, using a method well known to those skilled in the art. Additionally, the chemically synthesized oligopeptide may be chemically bonded to the N-terminal side or the C-terminal side of the Fc-binding protein.

Furthermore, a signal peptide for promoting efficient expression in a host may be added to the N-terminal side of the Fc-binding protein. Examples of the signal peptide in a case where the host is *Escherichia coli* include signal peptides that secrete a protein into the periplasm, such as PelB, DsbA, MalE (the region from position 1 to position 26 of the amino acid sequence of UniProt No. P0AEX9), TorT, and the like (JP2011-097898A). In particular,
(A) a polynucleotide encoding a natural OmpA signal peptide (the region from position 1 to position 21 of UniProt No. P0A910), or
(B) an oligonucleotide encoding the same polypeptide as the signal peptide described in (A) above except that one or several residues are subjected to (modified by) any one or more of substitutions, deletions, insertions, and additions is preferably used to allow more efficient production.

An example of a method of producing a polynucleotide encoding an Fc-binding protein of the present disclosure (hereinafter also simply referred to as the polynucleotide of the present disclosure) is
<1> a method of converting the amino acid sequence of the Fc-binding protein of the present disclosure into a nucleotide sequence, and artificially synthesizing a polynucleotide containing the nucleotide sequence, or
<2> a method of using a DNA amplification method such as a PCR method to prepare a polynucleotide containing the whole or a partial sequence of the Fc-binding protein directly artificially or from cDNA of the Fc-binding protein, and ligating the prepared polynucleotide by a suitable method.

When, in the method of <1>, conversion is performed from the amino acid sequence to the nucleotide sequence, it is preferable to perform the conversion considering codon usage in a host to be transformed. As an example, when the host is *Escherichia coli*, usage of AGA, AGG, CGG, and CGA is low for arginine (R), usage of ATA is low for isoleucine (I), usage of CTA is low for leucine (L), usage of GGA is low for glycine (G), and usage of CCC is low for proline (P) (because they are so-called rare codons), and thus, conversion may be performed so as to avoid these codons. Analysis of codon usage is also possible by using a public database (for example, the Codon Usage Database on the website of Kazusa DNA Research Institute or the like).

In a case where a host is transformed using a polynucleotide of the present disclosure, the polynucleotide itself may be used, but it is preferable to use an expression vector, such as, a bacteriophage, cosmid, plasmid, or the like, which is normally used for transformation of prokaryotic cells or eukaryotic cells, into which the polynucleotide is inserted at a suitable position. Note that the expression vector is not particularly limited as long as the expression vector exists stably and can replicate in the host to be transformed. In a case where *Escherichia coli* is used as the host, for example, a pET plasmid vector, a pUC plasmid vector, a pTrc plasmid vector, a pCDF plasmid vector, and a pBBR plasmid vector can be used. Additionally, the suitable position means a position at which a region related to the replication function, a desired antibiotic marker, and transmissibility of the expression vector is not destroyed. When inserted into the expression vector, the polynucleotide is preferably inserted in a state of being linked to a functional polynucleotide such as a promoter necessary for expression. In a case where the host is *Escherichia coli*, examples of the promoter include: a trp promoter, a tac promoter, a trc promoter, lac promoter, a T7 promoter, a recA promoter, and an lpp promoter; and furthermore, a λPL promoter and a λPR promoter of a λ phage.

To transform a host using an expression vector produced by the above method, into which expression vector the polynucleotide has been inserted and which expression vector contains the polynucleotide (hereinafter referred to as the expression vector of the present disclosure), a method normally used by those skilled in the art may be used. For example, in a case where a microorganism belonging to the genus *Escherichia* (an *Escherichia coli* JM109 strain, *Escherichia coli* BL21 (DE3) strain, *Escherichia coli* W3110 strain, or the like) is selected as the host, transformation may be performed by a method described in known literature (for example, Molecular Cloning, Cold Spring Harbor Laboratory, 256, 1992). The transformant obtained by transformation by the above-described method can be screened by a suitable method to obtain a transformant capable of expressing the Fc-binding protein (hereinafter referred to as a transformant of the present disclosure). Note that the host for expressing the Fc-binding protein is not particularly limited. Examples thereof include animal cells (CHO (Chinese Hamster Ovary) cells, HEK cells, Hela cells, COS cells, and the like), yeasts (*Saccharomyces cerevisiae*, *Pichia pastoris*, *Hansenula polymorpha*, *Schizosaccharomyces japonicus*, *Schizosaccharomyces octosporus*, *Schizosaccharomyces pombe*, and the like), insect cells (Sf9, Sf21, and the like), *Escherichia coli* (a JM109 strain, BL21 (DE3) strain, W3110 strain, and the like), and *Bacillus subtilis*. Note that an animal cell or *Escherichia coli* is preferably used as the host in terms of productivity, and *Escherichia coli* is more preferably used as the host.

To prepare an expression vector of the present disclosure from a transformant of the present disclosure, an alkali extraction method or a commercially available extraction kit such as a QIAprep Spin Miniprep kit (Qiagen N.V.) may be used to prepare the expression vector from a culture product obtained by culturing the transformant. The Fc-binding protein can be produced by culturing the transformant and collecting the Fc-binding protein from the obtained culture product. Note that in the present specification, the culture product may include not only the very cells of the cultured transformant of the present disclosure but also a medium used for the culture. The transformant used in the protein production method of the present disclosure may be cultured in a medium suitable for culture of a target host. In a case where the host is *Escherichia coli*, an LB (Luria-Bertani) medium supplemented with necessary nutrient sources is mentioned as an example of a preferable medium.

Note that in order to selectively proliferate a transformant of the present disclosure depending on the presence or absence of introduction of a vector of the present disclosure, it is preferable that an agent corresponding to a drug-resistance gene contained in the vector is added to the medium, and then the transformant is cultured. For example, in a case where the vector contains a kanamycin-resistance gene, kanamycin may be added to the medium. Additionally, in addition to carbon, nitrogen, and inorganic salt sources, a suitable nutrient source may be added to the medium. If desired, the culture medium may contain one or more reducing agents selected from the group consisting of glutathione, cysteine, cystamine, thioglycolate, and dithiothreitol. Furthermore, a reagent that promotes protein secretion from the transformant into the culture fluid, for example, glycine, may be added. Specifically, in a case where the host is *Escherichia coli*, glycine is preferably added at 2% (w/v) or less with respect to the medium.

In a case where the host is *Escherichia coli*, the culture temperature is generally 10°C to 40°C, preferably 20°C to 37°C, and more preferably approximately 25°C, but the culture temperature may be selected depending on the characteristics of a protein to be expressed. In a case where the host is *Escherichia coli*, the pH of the medium is pH 6.8 to pH 7.4, preferably approximately pH 7.0. In a case where a vector of the present disclosure contains an inducible promoter, induction is preferably performed under conditions where an Fc-binding protein of the present disclosure can be expressed well. Examples of the inducing agent include IPTG (isopropyl-β-D-thiogalactopyranoside). In a case where the host is *Escherichia coli*, expression of the Fc-binding protein can be induced by measuring the turbidity (absorbance at 600 nm) of the culture fluid, adding a suitable amount of IPTG when the turbidity reaches about 0.5 to 1.0, and subsequently performing culture. The concentration of IPTG to be added may be suitably selected from the range of from 0.005 mmol/L to 1.0 mmol/L, and the range of from 0.01 mmol/L to 0.5 mmol/L is preferable. The various conditions regarding IPTG induction may be conditions well known in the art.

In the present specification, "enhancement in productivity" means that the amount of the Fc-binding protein collectible from the same amount of the culture fluid is increased compared to the conventional amount.

To collect an Fc-binding protein of the present disclosure from a culture product obtained by culturing a transformant of the present disclosure, the Fc-binding protein may be collected by separating and purifying the Fc-binding protein from the culture product by a method suitable for an expression form of the Fc-binding protein in the transformant. For example, in a case where the Fc-binding protein is expressed in a culture supernatant, the bacterial cells may be separated by centrifugation, and the Fc-binding protein may be purified from the obtained culture supernatant. In a case where the Fc-binding protein is expressed in a cell (encompassing periplasm), the Fc-binding protein is preferably extracted by collecting the bacterial cells by centrifugation and then disrupting the bacterial cells by adding an enzyme treatment agent, a surfactant, or the like. Then, the Fc-binding protein may be purified. To purify the Fc-binding protein, a method known in the art may be used. Examples thereof include a separation and purification method based on liquid chromatography. Examples of the liquid chromatography include ion exchange chromatography, hydrophobic interaction chromatography, gel filtration chromatography, and affinity chromatography. The Fc-binding protein can be prepared with high purity by performing purification by combining these kinds of chromatographies. Evaluation of productivity of the Fc-binding protein may be performed by SDS-PAGE or absorbance measurement at 280 nm.

As a method of measuring the binding activity of the obtained Fc-binding protein to an antibody, for example, the binding activity for the antibody may be measured by an Enzyme-Linked Immunosorbent Assay (hereinafter referred to as ELISA) method. As the antibody, immunoglobulin G (IgG) is preferable. As the IgG used for measuring the binding activity, human IgG is preferable. Note that regarding the Fc-binding activity of the Fc-binding protein, the binding activity for not only the antibody but also a substance having an arbitrary Fc region may be measured by ELISA. Even the Fc-binding activity measured using a substance other than the antibody and having an arbitrary Fc region may substantially be considered to be activity that exhibits the binding activity for the antibody.

In the present specification, "enhanced alkali resistance" may mean that the binding activity of the Fc-binding protein treated with an alkali such as NaOH toward an antibody remains higher than the binding activity of a conventional Fc-binding protein.

The alkali resistance may be evaluated, for example, by treating the Fc-binding protein with an alkali and then measuring the binding activity for the antibody. The method of treating the Fc-binding protein with an alkali may be performed, for example, by allowing the Fc-binding protein to stand in a sodium hydroxide (NaOH) aqueous solution, more specifically, in a 0.01 mol/L to 0.05 mol/L NaOH aqueous solution. Examples of the method of measuring the binding activity of the Fc-binding protein to the antibody include the above-described method.

An antibody adsorbent can be produced by binding (immobilizing) an Fc-binding protein of the present disclosure to an insoluble carrier. The insoluble carrier is not particularly limited. Examples thereof include: a carrier the raw material for which is a polysaccharide, and which is, for example, agarose, alginate, carrageenan, chitin, cellulose, dextrin, dextran, or starch; a carrier the raw material for which is a synthetic polymer, and which is, for example, polyvinyl alcohol, polymethacrylate, poly(2-hydroxyethyl methacrylate), or polyurethane; and a carrier the raw material for which is a ceramic, and which is, for example, silica. Among the carriers, a carrier the raw material for which is a polysaccharide and a carrier the raw material for which is a synthetic polymer are preferable as the insoluble carrier. Examples of the preferable carrier include a polymethacrylate gel into which a hydroxy group has been introduced, such as Toyopearl (Tosoh Corporation); an agarose gel such as Sepharose (Cytiva); and a cellulose gel such as Cellufine (JNC Corporation). The shape of the insoluble carrier is not particularly limited, and may be any of a granular, non-granular shape, a porous or non-porous shape.

In order to immobilize an Fc-binding protein of the present disclosure to the insoluble carrier, immobilization may be performed by providing an active group such as an N-hydroxysuccinimide (NHS) activated ester group, epoxy group, carboxy group, maleimide group, haloacetyl group, tresyl group, formyl group, or haloacetamide (iodoacetamide, bromoacetamide, or the like) to an insoluble carrier, and covalently binding the human Fc-binding protein and the insoluble carrier via the active group. As the carrier provided with the active group, a commercially available carrier as is may be used, or the active group may be introduced into the surface of a carrier under suitable reaction conditions to prepare the carrier provided with the active group. Examples of the commercially available carrier provided with the active group include TOYOPEARL AF-Epoxy-650M, TOYOPEARL AF-Tresyl-650M (both from Tosoh Corporation), HiTrap NHS-activated HP Columns, NHS-activated Sepharose 4 Fast Flow, Epoxy-activated Sepharose 6B (all from Cytiva), and SulfoLink Coupling Resin (Thermo Fisher Scientific Inc.).

On the other hand, examples of a method of introducing the active group into the surface of the carrier include a method of allowing one of two or more active sites of a compound to react with a hydroxy group, epoxy group, carboxy group, amino group, or the like present in the surface of the carrier. Among examples of the compound, examples of a compound for introducing an epoxy group into the hydroxy group or the amino group in the surface of the carrier include epichlorohydrin, ethanediol diglycidyl ether, butanediol diglycidyl ether, and hexanediol diglycidyl ether. Examples of a compound for introducing a carboxy group into the surface of the carrier after introducing an epoxy group into the surface of the carrier using the above compound, include 2-mercaptoacetic acid, 3-mercaptopropionic acid, 4-mercaptobutyric acid, 6-mercaptobutyric acid, glycine, 3-aminopropionic acid, 4-aminobutyric acid, and 6-aminohexanoic acid.

Examples of a compound for introducing a maleimide group into a hydroxy group, an epoxy group, a carboxy group, or an amino group present in the surface of the carrier include N-(ε-maleimidocaproic acid) hydrazide, N-(ε-maleimidopropionic acid) hydrazide, 4-(4-N-maleimidophenyl) acetic acid hydrazide, 2-aminomaleimide, 3-aminomaleimide, 4-aminomaleimide, 6-aminomaleimide, 1-(4-aminophenyl) maleimide, 1-(3-aminophenyl) maleimide, 4-(maleimido) phenyl isocyanate, 2-maleimidoacetic acid, 3-maleimidopropionic acid, 4-maleimidobutyric acid, 6-maleimidohexanoic acid, N-(α-maleimidoacetoxy) succinimide ester, (m-maleimidobenzoyl) N-hydroxysuccinimide ester, succinimidyl-4-(maleimidomethyl) cyclohexane-1-carbonyl-6-aminohexanoic acid, succinimidyl-4-(maleimidomethyl) cyclohexane-1-carboxylic acid, (p-maleimidobenzoyl) N-hydroxysuccinimide ester, and (m-maleimidobenzoyl) N-hydroxysuccinimide ester.

Examples of a compound for introducing a haloacetyl group into a hydroxy group or an amino group present in the surface of the carrier include chloroacetic acid, bromoacetic acid, iodoacetic acid, chloroacetyl chloride, bromoacetyl chloride, bromoacetyl bromide, chloroacetic anhydride, bromoacetic anhydride, iodoacetic anhydride, 2-(iodoacetamido)acetic acid N-hydroxysuccinimide ester, 3-(bromoacetamido)propionic acid N-hydroxysuccinimide ester, and 4-(iodoacetyl)aminobenzoic acid N-hydroxysuccinimide ester. Note that a method of allowing ω-alkenylalkane glycidyl ether to react with a hydroxy group or an amino group present in the surface of the carrier and then halogenating and activating an ω-alkenyl site with a halogenating agent can also be an example. Examples of the ω-alkenylalkane glycidyl ether include allyl glycidyl ether, 3-butenyl glycidyl ether, and 4-pentenyl glycidyl ether. Examples of the halogenating agent include N-chlorosuccinimide, N-bromosuccinimide, and N-iodosuccinimide.

As another example of the method for introducing the active group into the surface of the carrier, there is a method of introducing an activating group into a carboxy group present in the surface of the carrier using a condensing agent and an additive. Examples of the condensing agent include 1-ethyl-3-(3-dimethylaminopropyl)carbodiimide (EDC), dicyclohexylcarbodiimide, and carbonyldiimidazole. Examples of the additive include N-hydroxysuccinimide (NHS), 4-nitrophenol, and 1-hydroxybenzotriazole.

Examples of a buffer used when an Fc-binding protein of the present disclosure is immobilized to an insoluble carrier include an acetic acid buffer, a phosphate buffer, a MES (2-morpholinoethanesulfonic acid) buffer, a HEPES (2-[4-(2-hydroxyethyl)-1-piperazinyl]ethanesulfonic acid) buffer, a Tris buffer, a boric acid buffer, and a bistrispropane (1,3-bis[tris(hydroxymethyl)methylamino]propane) buffer. The reaction temperature during the immobilization may be suitably set within a temperature range of 5°C or higher and 50°C or lower in consideration of the reactivity of the active group and the stability of the Fc-binding protein and is preferably in a range of 10°C or higher and 35°C or lower.

In order to purify an antibody using an antibody adsorbent obtained by immobilizing an Fc-binding protein of the present disclosure to an insoluble carrier, for example, the antibody may be eluted by using a liquid feeding means such as a pump to add a buffer containing the antibody to a column packed with the adsorbent, thereby allowing the adsorbent to specifically adsorb the antibody, and then adding a suitable eluent to the column.

By using the adsorbent, a substance having an Fc region can be separated. Examples of the substance having an Fc region include an antibody, an Fc fusion protein, and a complex of an Fc region and a drug. The antibody is not particularly limited to any aspect as long as the antibody contains at least an Fc region of an antibody having affinity for the Fc-binding protein. Examples of the antibody include a chimeric antibody, a humanized antibody, a human antibody, an amino acid substitution product thereof, and a bispecific antibody, which are generally used as antibodies for antibody drugs. Additionally, examples of the Fc fusion protein include a protein obtained by fusing a peptide, a nucleic acid, or a protein with an Fc region. Examples of the complex of an Fc region of an antibody and a drug include an antibody-drug complex (antibody-drug conjugate: ADC). The Fc region of the substance having the Fc region may contain at least an Fc region of IgG. Additionally, the Fc region of the substance having the Fc region may contain at least an Fc region of human IgG.

Specifically, the Fc region in the present specification may mean a region having the amino acid sequence of any one of SEQ ID NOs: 101 to 117 in a protein, may mean a region having the same amino acid sequence as any one of SEQ ID NOs: 101 to 117 except that any one or more of substitutions, deletions, insertions, and additions of one or several amino acid residues at one or several positions are introduced, or may mean a region having an amino acid sequence having an identity of 70% or more, 80% or more, 85% or more, or 90% or more to the amino acid sequence of any one of SEQ ID NOs: 101 to 117. Note that "one or several" varies depending on the position of the amino acid residue in the three-dimensional structure of the protein and the species of the amino acid residue, but may mean, for example, any one of 1 to 50, 1 to 40, 1 to 35, 1 to 30, 1 to 25, 1 to 20, 1 to 15, 1 to 10, 1 to 9, 1 to 8, 1 to 7, 1 to 6, 1 to 5, 1 to 4, 1 to 3, and 1 to 2.

Examples of the substance having a region having the amino acid sequence of any one of SEQ ID NOs: 101 to 117 include Rituxan, Synagis, Avastin, Erbitux, Herceptin, Kadcyla, Remicade, Humira, Actemra, Vectibix, Ranmark, Emgality, Zaltrap, Trulicity, Orencia, Romiplate, and Ultomiris. In other words, these substances may represent an example of the substance having an Fc region. Note that the amino acid sequence of SEQ ID NO: 117 is the amino acid sequence of the Fc region possessed by Ultomiris.

Additionally, it is preferable to equilibrate the column using an appropriate buffer before adding the buffer containing the antibody to the column, because the equilibration enables the antibody to be purified with higher purity. Examples of the buffer include a buffer containing an inorganic salt as a component, such as a phosphate buffer. The pH of the buffer is pH 3.0 or higher and 10.0 or lower, and preferably pH 5.0 or higher and 8.0 or lower.

In order to elute the antibody adsorbed to the adsorbent, the interaction between the antibody and the ligand (an Fc-binding protein of the present disclosure) may be weakened. Specifically, the interaction between the antibody and the ligand may be weakened by, for example, a buffer-based pH change, a counter peptide, a temperature change, or a salt concentration change. Specific examples of the eluent for eluting the antibody adsorbed to the adsorbent include a buffer on a more basic side than the solution used when the antibody is adsorbed to the adsorbent. Examples of the species of the buffer include a phosphate buffer having a buffering capacity from neutral to basic, a HEPES (2-[4-(2-hydroxyethyl)-1-piperazinyl]ethanesulfonic acid) buffer, a Tris buffer, a boric acid buffer, and a bistrispropane (1,3-bis[tris(hydroxymethyl)methylamino]propane) buffer. The pH of the buffer may be set within a range that does not impair the function possessed by the antibody, and may be preferably pH 7.0 or higher and 10.0 or lower, and more preferably pH 7.0 or higher and 9.0 or lower.

According to one aspect, an Fc-binding protein of the present disclosure has excellent alkali resistance. Accordingly, any buffer used for the antibody adsorbent obtained by immobilizing an Fc-binding protein of the present disclosure to an insoluble carrier may have a high pH without particular limitation. Additionally, even if the pH of the buffer is high, the adsorbent may be reused.

### Examples

Hereinafter, the present disclosure will be described in detail with reference to Examples and Comparative Examples, but the present disclosure is not limited to these Examples.

### Example 1: Preparation of plasmid vector (pETMalE-p7)

A plasmid vector was prepared by the below-described method.
(1) A polynucleotide encoding a MalE signal peptide (an oligopeptide composed of 26 residues on the N-terminal side of UniProt No. P0AEX9, SEQ ID NO: 3) was prepared by the below-described two-step PCR.
(1-1) Based on the reaction liquid composition in Table 1, a first stage PCR was performed by repeating 5 cycles of a reaction consisting of a first step at 98°C for 10 seconds, a second step at 55°C for 5 seconds, and a third step at 72°C for 1 minute per cycle. Note that as the oligonucleotides, oligonucleotides consisting of the base sequences of SEQ ID NO: 4 (5'-TATACATATGAAAATAAAAACAGGTGCACGCATCC-3'), SEQ ID NO: 5 (5'-GCATTAACGACGATGATGTTTTCCGCCTCGGCTCTCGCC-3'), SEQ ID NO: 6 (5'-ATCGTCGTTAATGCGGATAATGCGAGGATGCGTGCACCTG-3'), and SEQ ID NO: 7 (5'-TTGTCCCATGGCTTCTTCGATTTTGGCGAGAGCCG-3') were used.

### [Table 1]

**Table 1**

| Composition | Volume |
|---|---|
| Oligonucleotides (SEQ ID NOs: 4-7) | 2.5 µM each |
| dNTPs mixture | 0.2 mM each |
| PrimeSTAR HS polymerase (Takara Bio Inc.) | 0.025 U/µL |
| 5 × PrimeSTAR Buffer (Takara Bio Inc.) | ×1 |

(1-2) Based on the reaction liquid composition in Table 2, a second stage PCR was performed by repeating 30 cycles of a reaction consisting of a first step at 98°C for 10 seconds, a second step at 55°C for 5 seconds, and a third step at 72°C for 1 minute per cycle. Note that the first stage PCR product obtained in (1-1) was used as the template DNA, and the oligonucleotide (forward primer) consisting of the base sequence of SEQ ID NO: 8 (5'-TATACATATGAAAATAAAAACAGGTGCACGCATCC-3') and the oligonucleotide (reverse primer) consisting of the base sequence of SEQ ID NO: 9 (5'-GCATTAACGACGATGATGTTTTCCGCCTCGGCTCTCGCC-3') were used as the PCR primers, respectively.

### [Table 2]

**Table 2**

| Composition | Volume |
|---|---|
| Template DNA | 2.5 µM each |
| PCR forward primer | 0.4 µM |
| PCR reverse primer | 0.4 µM |
| dNTPs mixture | 0.2 mM each |
| PrimeSTAR HS polymerase (Takara Bio Inc.) | 0.025 U/µL |
| 5 × PrimeSTAR Buffer (Takara Bio Inc.) | ×1 |

(2) The polynucleotide encoding the MalE signal peptide, prepared in (1), was digested with restriction enzymes NdeI and NcoI, and then ligated into a plasmid vector pET-26b(+) (Novagen) previously digested with restriction enzymes NdeI and NcoI. An *Escherichia coli* BL21(DE3) strain (Novagen) was transformed with the resultant plasmid vector, using the calcium chloride method.
(3) The obtained transformant was cultured in LB (Luria-Bertani) medium containing 50 µg/mL kanamycin sulfate (Nacalai Tesque, Inc.). Then, a QIAprep Spin Miniprep kit (Qiagen N.V.) was used to extract a plasmid vector (named pETMalE).
(4) PCR was performed in the same manner as in (1-2) except that the plasmid vector pETMalE obtained in (3) was used as the template DNA, and that the oligonucleotide (forward primer) consisting of the base sequence of SEQ ID NO: 10 (5'-AGTAGTAGGTTGAGGCCGTTGAG-3') and the oligonucleotide (reverse primer) consisting of the base sequence of SEQ ID NO: 11 (5'-TTTTCATATGTATTATGTATATCTCCTTCTTAAA-3') were used as the PCR primers, respectively.
(5) The PCR product obtained in (4) was digested with restriction enzymes SphI and NdeI, and then ligated into pETMalE previously digested with restriction enzymes SphI and NdeI. Using the resultant product, an *Escherichia coli* BL21(DE3) strain was transformed.
(6) After culturing the transformant of (5), the plasmid was extracted (named pETMalE-p7).

### Example 2: Preparation of Fc-binding protein expression plasmid (MalE)

A polynucleotide encoding the Fc-binding protein FcRn(B2M-GS-EC_m7a)-6H consisting of the amino acid sequence of SEQ ID NO: 12 was inserted into the expression vector pETMalE-p7 prepared in Example 1, using the below-described method, to prepare a plasmid capable of expressing the protein. Note that in FcRn(B2M-GS-EC_m7a)-6H, positions 1 to 99 are the β2 microglobulin region (the region from positions 21 to 119 of SEQ ID NO: 2, B2M) of human FcRn β-chain, positions 100 to 124 are the GS linker sequence (a sequence in which an oligopeptide consisting of 4 glycine (G) residues and 1 serine (S) residue is repeated 5 times, GS), positions 125 to 398 are the amino acid substitution product region (named EC_m7a, SEQ ID NO: 13) of the extracellular region of human FcRn α-chain, and positions 399 to 404 are the histidine tag (6H) sequence. Additionally, EC_m7a is a polypeptide obtained by introducing the following 7 amino acid substitutions into the extracellular region (region from positions 24 to 297 of SEQ ID NO: 1, EC) of human FcRn α-chain:
cysteine (C) at position 71 of SEQ ID NO: 1 (position 48 in SEQ ID NO: 13) is substituted with arginine (R);
asparagine (N) at position 78 of SEQ ID NO: 1 (position 55 in SEQ ID NO: 13) is substituted with aspartic acid (D);
glycine (G) at position 151 of SEQ ID NO: 1 (position 128 in SEQ ID NO: 13) is substituted with aspartic acid (D);
arginine (R) at position 192 of SEQ ID NO: 1 (position 169 in SEQ ID NO: 13) is substituted with leucine (L);
asparagine (N) at position 196 of SEQ ID NO: 1 (position 173 in SEQ ID NO: 13) is substituted with aspartic acid (D);
glutamine (Q) at position 232 of SEQ ID NO: 1 (position 209 in SEQ ID NO: 13) is substituted with leucine (L); and
lysine (K) at position 295 of SEQ ID NO: 1 (position 272 in SEQ ID NO: 13) is substituted with glutamic acid (E).

(1) In order that a cysteine tag (SEQ ID NO: 15) for immobilization to an insoluble carrier could be added to the C-terminal side of FcRn(B2M-GS-EC_m7a)-6H, PCR was performed in the same manner as in Example 1 (1-2) except that a plasmid containing a polynucleotide (SEQ ID NO: 14) encoding the Fc-binding protein consisting of the amino acid sequence of SEQ ID NO: 12 was used as the template DNA, and that the oligonucleotide (forward primer) consisting of the base sequence of SEQ ID NO: 16 (5'-GCCTCGGCTCTCGCCATTCAACGTACGCCAAAAATC-3') and the oligonucleotide (reverse primer) consisting of the base sequence of SEQ ID NO: 17 (5'-AGTGCGGCCGCAAGCTTATCCGCAGGTATCGTTGCGGCAGTGATGATGAT GATGATGAGAGGATTCGGC-3') were used as the PCR primers, respectively.
(2) The PCR product obtained in (1) was subjected to agarose gel electrophoresis, and purification was performed from the gel using a QIAquick Gel Extraction kit (Qiagen N.V.).
(3) PCR was performed in the same manner as in Example 1 (1-2) except that pETMalE-p7 prepared in Example 1 was used as the template DNA, and that the oligonucleotide (forward primer) consisting of the base sequence of SEQ ID NO: 18 (5'-GCTTGCGGCCGCACTCGAGCACCACCACCACCACCACTGAGA-3') and the oligonucleotide (reverse primer) consisting of the base sequence of SEQ ID NO: 19 (5'-GGCGAGAGCCGAGGCGGAAAACATCATCGTCGTTAA-3') were used as the PCR primers, respectively.
(4) The PCR product obtained in (3) was subjected to agarose gel electrophoresis, and purification was performed using the method described in (2).
(5) The purified PCR products obtained in (2) and (4) were ligated using InFusion HD Cloning Kit (Takara Bio Inc.), and then, the *Escherichia coli* JM109 strains (Takara Bio Inc.) were transformed using the ligation products.
(6) From the obtained transformants, plasmids (named pETMalE-FcRn(B2M-GS-EC_m7a)-6HC-p7) were extracted using the method described in Example 1 (3).
(7) In the plasmids pETMalE-FcRn(B2M-GS-EC_m7a)-6HC-p7 extracted in (6), the base sequences of the polynucleotides encoding the MalE signal peptide and FcRn(B2M-GS-EC_m7a) and of the surrounding regions thereof were analyzed using a fully automatic DNA sequencer Genetic Analyzer 3500 (Thermo Fisher Scientific Inc.), and the sequences were confirmed to be the desired sequences. Note that in the analysis, the oligonucleotides consisting of the sequences of SEQ ID NO: 20 (5'-GGATCTCGACGCTCTCCCT-3'), SEQ ID NO: 21 (5'-ATGGGCATTCAACGTACGCCAAA-3'), SEQ ID NO: 22 (5'-CTGCGCATCAAGGAAAAACTGTTC-3'), SEQ ID NO: 23 (5'-CACGCTGGTCTCGCGCAACC-3'), and SEQ ID NO: 24 (5'-ATGCTAGTTATTGCTCAGCGG-3') were used as sequencing primers.

The amino acid sequence of the polypeptide MalE-FcRn(B2M-GS-EC_m7a)-6HC expressed by the plasmid pETMalE-FcRn(B2M-GS-EC_m7a)-6HC-p7 is shown in SEQ ID NO: 25, and the base sequence of the polynucleotide encoding the polypeptide is shown in SEQ ID NO: 26, respectively. Note that in SEQ ID NO: 25, methionine (M) at position 1 to alanine (A) at position 26 is the MalE signal peptide (SEQ ID NO: 3), isoleucine (I) at position 27 to methionine (M) at position 125 is the B2M region, glycine (G) at position 126 to serine (S) at position 150 is the GS linker sequence, alanine (A) at position 151 to serine (S) at position 424 is EC_m7a (SEQ ID NO: 13), histidine (H) at positions 425 to 430 is the histidine tag sequence, and cysteine (C) at position 431 to glycine (G) at position 437 is the cysteine tag sequence (SEQ ID NO: 15).

### Example 3: Preparation of Fc-binding protein expression plasmid (OmpA)

A polypeptide (SEQ ID NO: 28) was designed in which the MalE signal peptide (SEQ ID NO: 3) in MalE-FcRn(B2M-GS-EC_m7a)-6HC (SEQ ID NO: 25) was substituted with the OmpA signal peptide (an oligopeptide consisting of 21 residues on the N-terminal side of UniProt No. P0A910, SEQ ID NO: 27), and a plasmid capable of expressing the polypeptide was prepared.
(1) PCR was performed in the same manner as in Example 1 (1-2) except that the plasmid pETMalE-FcRn(B2M-GS-EC_m7a)-6HC-p7 prepared in Example 2 was used as the template DNA, and that the oligonucleotide (forward primer) consisting of the base sequence of SEQ ID NO: 29 (5'-GCTGGTTTCGCTACCGTAGCGCAGGCCATTCAACGTACGCCAAAAATCCA AGTATACTCACG-3') and the oligonucleotide (reverse primer) consisting of the base sequence of SEQ ID NO: 30 (5'-GGTAGCGAAACCAGCCAGTGCCACTGCAATCGCGATAGCTGTCTTTTTCA TATGTATTATGTATATC-3') were used as the PCR primers, respectively.
(2) The PCR product obtained in (1) was subjected to agarose gel electrophoresis, and purification was performed using the method described in Example 2 (2).
(3) The purified PCR product obtained in (2) was subjected to ligation and transformation by the method described in Example 2 (5), and the obtained transformant was cultured by the method described in Example 2 (6) to prepare a plasmid (named pETOmpA-FcRn(B2M-GS-EC_m7a)-6HC-p7).
(4) In the plasmid pETOmpA-FcRn(B2M-GS-EC_m7a)-6HC-p7 prepared in (3), the base sequences of the polynucleotides encoding the OmpA signal peptide and FcRn(B2M-GS-EC_m7a), and of the surrounding regions thereof were analyzed by the method described in Example 2 (7), and the sequences were confirmed to be the desired sequences.

The amino acid sequence of the polypeptide expressed by the plasmid pETOmpA-FcRn(B2M-GS-EC_m7a)-6HC-p7 is shown in SEQ ID NO: 28, and the base sequence of the polynucleotide encoding the polypeptide is shown in SEQ ID NO: 31, respectively. Note that in SEQ ID NO: 28, methionine (M) at position 1 to alanine (A) at position 21 is the OmpA signal peptide (SEQ ID NO: 27), isoleucine (I) at position 22 to methionine (M) at position 120 is the B2M region, glycine (G) at position 121 to serine (S) at position 145 is the GS linker sequence, alanine (A) at position 146 to serine (S) at position 419 is EC_m7a (SEQ ID NO: 13), histidine (H) at positions 420 to 425 is the histidine tag sequence, and cysteine (C) at position 426 to glycine (G) at position 432 is the cysteine tag sequence (SEQ ID NO: 15).

### Example 4: Site-specific amino acid substitution to Fc-binding protein

For EC_m7a, site-specific amino acid substitution was performed on cysteine at position 251 of SEQ ID NO: 13 (position 274 in SEQ ID NO: 1, position 396 in SEQ ID NO: 28) (the cysteine is hereinafter also referred to as "Cα274").
(1) PCR was performed in the same manner as in Example 1 (1-2) except that the plasmid pETOmpA-FcRn(B2M-GS-EC_m7a)-6HC-p7 prepared in Example 3 was used as the template DNA, and that a combination of oligonucleotides consisting of the sequences shown in SEQ ID NOs in Table 3 was used as PCR primers for amino acid substitution, respectively.

### [Table 3]

**Table 3**

| Fc-binding protein | | PCR primer [SEQ ID NO] | |
|---|---|---|---|
| Amino acid substitution of Cα274 | SEQ ID NO: | Forward | Reverse |
| A | 32 | 33 | 34 |
| D | 35 | 36 | 37 |
| E | 38 | 39 | 40 |
| F | 41 | 42 | 43 |
| G | 44 | 45 | 46 |
| H | 47 | 48 | 49 |
| I | 50 | 51 | 52 |
| K | 53 | 54 | 55 |
| L | 56 | 57 | 58 |
| M | 59 | 60 | 61 |
| N | 62 | 63 | 64 |
| P | 65 | 66 | 67 |
| Q | 68 | 69 | 70 |
| R | 71 | 72 | 73 |
| S | 74 | 75 | 76 |
| T | 77 | 78 | 79 |
| V | 80 | 81 | 82 |
| W | 83 | 84 | 85 |
| Y | 86 | 87 | 88 |

(2) After preparing a plasmid by the method described in Example 3 (2) to (3), the base sequences of the amino acid substitution introduction site and the surrounding regions thereof were analyzed by the method described in Example 2 (7), and the sequences were confirmed to be the desired sequences.

### Example 5: Expression of Fc-binding protein (Part 1)

The Fc-binding protein was expressed by the below-described method.
(1) The transformant capable of expressing the Fc-binding protein, obtained by transforming the *Escherichia coli* BL21(DE3) strain with the plasmid prepared in Example 3 or Example 4, was inoculated into 10 mL of TB liquid medium (12 g/L Tryptone, 24 g/L Yeast Extract, 10 g/L glycerol, 9.4 g/L dipotassium hydrogen phosphate, and 2.2 g/L potassium dihydrogen phosphate) containing 50 µg/mL kanamycin, and preculture was performed by shaking culture aerobically at 37°C overnight.
(2) In a 1 L baffled flask, 3 mL of the preculture fluid of (1) was inoculated into 200 mL of TB liquid medium supplemented with 50 µg/mL kanamycin, and shaking culture was performed aerobically at 30°C.
(3) Two hours after the start of culture, IPTG (isopropyl β-D-1-thiogalactopyranoside) was added to a final concentration of 0.05 mmol/L while cooling on ice, and subsequently, expression was induced by shaking culture aerobically at 25°C overnight.
(4) After completion of the culture, bacterial cells were collected by centrifuging the culture fluid at 4°C at 8000 rpm for 20 minutes.
(5) After collecting the bacterial cells from the culture fluid of the transformant, 100 mmol/L Tris-HCl buffer (pH 8.0) containing 150 mmol/L sodium chloride, 2.4 mmol/L magnesium sulfate, 5000 Unit/L Benzonase (Merck KGaA), 0.006% (w/v) lysozyme, and 0.6% (w/v) Triton X-100 (trade name) was added. The resultant mixture was left to stand at room temperature for 2 hours to extract the bacterial cells. Then, centrifugation was performed at 4°C at 15000 rpm for 20 minutes to obtain an extract containing the expressed Fc-binding protein.
(6) The extract obtained in (5) was applied to an Econo-Pac column (Bio-Rad Laboratories, Inc.) packed with 1 mL of Ni-NTA agarose (Fujifilm Wako Pure Chemical Corporation) equilibrated in advance with 100 mmol/L Tris buffer (pH 8.0) containing 150 mmol/L sodium chloride.
(7) After washing the column with 100 mmol/L Tris-HCl buffer (pH 8.0) containing 20 mmol/L imidazole and 150 mmol/L sodium chloride, a purified solution containing the Fc-binding protein was obtained by elution with 100 mmol/L Tris buffer (pH 8.0) containing 300 mmol/L imidazole and 150 mmol/L sodium chloride.
(8) The eluted fraction (purified solution) obtained in (7) was subjected to SDS-PAGE (sodium dodecyl sulfate-polyacrylamide gel electrophoresis), and stained with a CBB (Coomassie Brilliant Blue) staining solution (Fujifilm Wako Pure Chemical Corporation). Then, the band intensity corresponding to the Fc-binding protein was quantified using image analysis software (ImageQuant TL 10.0, Cytiva).

The results are shown in FIG. 3. Note that in FIG. 3, the band intensity is expressed as a relative value, assuming that the value in the unsubstituted state (that is, Cα274 as is) set to 1. It can be seen that substitution of Cα274 with another amino acid increases the band intensity, that is, enhances the expression level of the Fc-binding protein. In particular, it can be seen that substituting Cα274 with any of glutamic acid (E) (Cα274E, SEQ ID NO: 38), histidine (H) (Cα274H, SEQ ID NO: 47), and serine (S) (Cα274S, SEQ ID NO: 74) remarkably enhances the expression level of the Fc-binding protein (Cα274E: 5.97 times, Cα274H: 4.72 times, and Cα274S: 4.70 times).

### Comparative Example 1

For the Fc-binding protein OmpA-FcRn(B2M-GS-EC_m7a)-6HC, a polypeptide (named OmpA-FcRn(B2M-GS-EC_m6)-6HC, SEQ ID NO: 89) was designed in which aspartic acid (D) at position 273 of SEQ ID NO: 28 (position 151 in SEQ ID NO: 1) was returned to the natural sequence (glycine (G)), and a plasmid capable of expressing the polypeptide was prepared.
(1) PCR was performed in the same manner as in Example 1 (1-2) except that the plasmid pETOmpA-FcRn(B2M-GS-EC_m7a)-6HC-p7 prepared in Example 3 was used as the template DNA, and that the oligonucleotide (forward primer) consisting of the base sequence of SEQ ID NO: 90 (5'-CCTGGGGCGGCGATTGGCCAGAGGC-3') and the oligonucleotide (reverse primer) consisting of the base sequence of SEQ ID NO: 91 (5'-TCGCCGCCCCAGGTGCCCTGTTTCA-3') were used as the PCR primers, respectively.
(2) After preparing a plasmid (named pETOmpA-FcRn(B2M-GS-EC_m6)-6HC-p7) by the method described in Example 3 (2) to (3), the base sequences of the amino acid substitution introduction site and the surrounding regions thereof were analyzed by the method described in Example 2 (7), and the sequences were confirmed to be the desired sequences.

### Example 6: Site-specific amino acid substitution to OmpA-FcRn(B2M-GS-EC_m6)-6HC

From the results of site-specific amino acid substitution to Cα274 (FIG. 3), substitution with serine (Cα274S) was selected from among the amino acid substitutions that remarkably enhanced the expression level of the Fc-binding protein, and a plasmid for expressing an Fc-binding protein (named OmpA-FcRn(B2M-GS-EC_m7b)-6HC, SEQ ID NO: 92) in which the amino acid substitution was introduced into OmpA-FcRn(B2M-GS-EC_m6)-6HC (SEQ ID NO: 89) was prepared.
(1) PCR was performed in the same manner as in Example 1 (1-2) except that the plasmid pETOmpA-FcRn(B2M-GS-EC_m6)-6HC-p7 prepared in Comparative Example 1 was used as the template DNA, and that the oligonucleotide (forward primer) consisting of the base sequence of SEQ ID NO: 75 (5'-ACTACAGCTGCATCGTGCAGCACGC-3') and the oligonucleotide (reverse primer) consisting of the base sequence of SEQ ID NO: 76 (5'-ATGCAGCTGTAGTGATGTTCGTCGC-3') were used as the PCR primers, respectively.
(2) After preparing a plasmid (named pETOmpA-FcRn(B2M-GS-EC_m7b)-6HC-p7) by the method described in Example 3 (2) to (3), the base sequences of the amino acid substitution introduction site and the surrounding regions thereof were analyzed by the method described in Example 2 (7), and the sequences were confirmed to be the desired sequences.

### Example 7: Expression of Fc-binding protein (Part 2)

The Fc-binding protein was expressed by the below-described method.
(1) Transformants capable of expressing the Fc-binding protein, obtained by transforming an *Escherichia coli* BL21(DE3) strain with each of the plasmid capable of expressing OmpA-FcRn(B2M-GS-EC_m7a)-6HC (SEQ ID NO: 28) and prepared in Example 3, the plasmid capable of expressing the polypeptide consisting of the sequence described in SEQ ID NO: 74 and prepared in Example 4, the plasmid capable of expressing OmpA-FcRn(B2M-GS-EC_m6)-6HC (SEQ ID NO: 89) and prepared in Comparative Example 1, and the plasmid capable of expressing OmpA-FcRn(B2M-GS-EC_m7b)-6HC (SEQ ID NO: 92) and prepared in Example 6, were inoculated into 30 mL of TB liquid medium containing 50 µg/mL kanamycin. Then, preculture was performed by shaking culture aerobically at 37°C overnight.
(2) In a 5 L baffled flask, 30 mL of the preculture fluid of (1) was inoculated into 1 L of TB liquid medium supplemented with 50 µg/mL kanamycin, and shaking culture was performed aerobically at 30°C.
(3) Expression induction by IPTG was applied by the method described in Example 5 (3), and bacterial cells (transformants) were collected by the method described in Example 5 (4).
(4) After collecting the bacterial cells from the culture fluid of the transformant, 50 mmol/L Bis-Tris propane buffer (pH 10.0) containing 150 mmol/L sodium chloride, 2.4 mmol/L magnesium sulfate, 5000 Unit/L Benzonase (Merck KGaA), 0.006% (w/v) lysozyme, and 0.6% (w/v) Triton X-100 (trade name) was added. By leaving the extract to stand at room temperature for 2 hours, bacterial cells were extracted. Then, centrifugation was performed at 4°C at 15000 rpm for 20 minutes to obtain an extract containing the expressed Fc-binding protein.
(5) The extract containing the Fc-binding protein, obtained in (4), was applied to an Econo-Pac column (Bio-Rad Laboratories, Inc.) packed with 5 mL of Ni-NTA agarose (Fujifilm Wako Pure Chemical Corporation) equilibrated in advance with 50 mmol/L Bis-Tris propane buffer (pH 10.0) containing 150 mmol/L sodium chloride. After washing with 50 mmol/L Bis-Tris propane buffer (pH 10.0) containing 20 mmol/L imidazole and 150 mmol/L sodium chloride, a crudely purified solution containing the Fc-binding protein was obtained by elution with 50 mmol/L Bis-Tris propane buffer (pH 10.0) containing 300 mmol/L imidazole and 150 mmol/L sodium chloride.
(6) The crudely purified solution obtained in (5) was adjusted to pH 6.5 using 1 M hydrochloric acid (Fujifilm Wako Pure Chemical Corporation), and then applied to a Poly-Prep column (Bio-Rad Laboratories, Inc.) packed with 2 mL of IgG-Sepharose (Cytiva) equilibrated in advance with 50 mmol/L Bis-Tris propane buffer (pH 6.5) containing 150 mmol/L sodium chloride. After washing with the buffer used for equilibration, a purified Fc-binding protein was obtained by elution with 50 mmol/L Bis-Tris propane buffer (pH 8.5) containing 150 mmol/L sodium chloride. Note that the purified Fc-binding protein obtained using the plasmid capable of expressing OmpA-FcRn(B2M-GS-EC_m7a)-6HC (SEQ ID NO: 28) is also referred to as FcRnm7a (corresponding to isoleucine (I) at position 22 to glycine (G) at position 432 of SEQ ID NO: 28). Additionally, the purified Fc-binding protein obtained using the plasmid capable of expressing OmpA-FcRn(B2M-GS-EC_m7b)-6HC (SEQ ID NO: 92) is also referred to as FcRnm7b (corresponding to isoleucine (I) at position 22 to glycine (G) at position 432 of SEQ ID NO: 92).
(7) The absorbance (wavelength: 280 nm) of the purified protein solution obtained in (6) was measured to calculate the purification yield.

The results of comparing the purification yields are shown in Table 4 and Table 5. Note that in Table 4 and Table 5, the purification yield is expressed as a relative value, assuming that the purification yield of the Fc-binding protein into which the Cα274S amino acid substitution was not introduced is taken as 1.00. In any case, it can be seen that the purification yield is enhanced by introducing the Cα274S amino acid substitution.

### [Table 4]

**Table 4**

| SEQ ID NO | Cα274S amino acid substitution | Purification yield (Relative value) |
|---|---|---|
| 28 | None | 1 |
| 74 | Introduced | 1.31 |

### [Table 5]

**Table 5**

| SEQ ID NO | Ca274S amino acid substitution | Purification yield (Relative value) |
|---|---|---|
| 89 | None | 1 |
| 92 | Introduced | 1.21 |

### Example 8: Introduction of amino acid substitution into OmpA-FcRn(B2M-GS-EC_m7b)-6HC

A plasmid for expressing an Fc-binding protein (named OmpA-FcRn(B2M-GS-EC_m10)-6HC, SEQ ID NO: 93) obtained by introducing three amino acid substitutions into the Fc-binding protein OmpA-FcRn(B2M-GS-EC_m7b)-6HC (SEQ ID NO: 92) as described below was prepared.

Valine (V) at position 202 of SEQ ID NO: 92 (position 80 in SEQ ID NO: 1) was substituted with aspartic acid (D) (the substitution is hereinafter also referred to as "Vα80D"),

Lysine (K) at position 218 of SEQ ID NO: 92 (position 96 in SEQ ID NO: 1) was substituted with glutamic acid (E) (the substitution is hereinafter also referred to as "Kα96E"), and

Asparagine (N) at position 294 of SEQ ID NO: 92 (position 172 in SEQ ID NO: 1) was substituted with aspartic acid (D) (the substitution is hereinafter also referred to as "Nα172D").
(1) PCR was performed in the same manner as in Example 1 (1-2) except that the plasmid pETOmpA-FcRn(B2M-GS-EC_m7b)-6HC-p7 prepared in Example 6 was used as the template DNA, and that the oligonucleotide (forward primer) consisting of the base sequence of SEQ ID NO: 94 (5'-ACCAGGATTCTTGGTACTGGGAGAA-3') and the oligonucleotide (reverse primer) consisting of the base sequence of SEQ ID NO: 95 (5'-CAAGAATCCTGGTCTTCCCAAACCC-3') were used as the PCR primers, respectively.
(2) A plasmid (named pETOmpA-FcRn(B2M-GS-EC_m8)-6HC-p7) was prepared by the method described in Example 3 (2) to (3).
(3) PCR was performed in the same manner as in Example 1 (1-2) except that pETOmpA-FcRn(B2M-GS-EC_m8)-6HC-p7 prepared in (2) was used as the template DNA, and that the oligonucleotide (forward primer) consisting of the base sequence of SEQ ID NO: 96 (5'-AGGAAGAACTGTTCCTGGAAGCCTT-3') and the oligonucleotide (reverse primer) consisting of the base sequence of SEQ ID NO: 97 (5'-AACAGTTCTTCCTTGATGCGCAGAT-3') were used as the PCR primers, respectively.
(4) A plasmid (named pETOmpA-FcRn(B2M-GS-EC_m9)-6HC-p7) was prepared by the method described in Example 3 (2) to (3).
(5) PCR was performed in the same manner as in Example 1 (1-2) except that pETOmpA-FcRn(B2M-GS-EC_m9)-6HC-p7 prepared in (4) was used as the template DNA, and that the oligonucleotide (forward primer) consisting of the base sequence of SEQ ID NO: 98 (5'-CTGCGGATAAAGAACTGACGTTCCT-3') and the oligonucleotide (reverse primer) consisting of the base sequence of SEQ ID NO: 99 (5'-TCTTTATCCGCAGCCTTATCTTGCT-3') were used as the PCR primers, respectively.
(6) After preparing a plasmid (named pETOmpA-FcRn(B2M-GS-EC_m10)-6HC-p7) by the method described in Example 3 (2) to (3), the base sequences of the amino acid substitution introduction site and the surrounding regions thereof were analyzed by the method described in Example 2 (7), and the sequences were confirmed to be the desired sequences (that is, the polynucleotide (SEQ ID NO: 100) encoding OmpA-FcRn(B2M-GS-EC_m10)-6HC (SEQ ID NO: 93) was inserted into the plasmid).

### Example 9: Expression of Fc-binding protein (Part 3)

The Fc-binding protein was expressed by the below-described method.
(1) Recombinant *Escherichia coli* capable of expressing an Fc-binding protein, obtained by transforming an *Escherichia coli* W3110 strain with a plasmid capable of expressing OmpA-FcRn(B2M-GS-EC_m7b)-6HC (SEQ ID NO: 92) and prepared in Example 6 or a plasmid capable of expressing OmpA-FcRn(B2M-GS-EC_m10)-6HC (SEQ ID NO: 93) and prepared in Example 8, was inoculated into a 2×YT medium (tryptone: 16 g/L, yeast extract: 10 g/L, sodium chloride: 5 g/L, and kanamycin sulfate: 50 mg/L). Preculture was performed at 30°C for 16 hours.
(2) The main culture was performed in 36 mL of the preculture fluid of (1) supplemented with 1.2 L of the main culture initial medium (18 g/L disodium hydrogen phosphate dodecahydrate, 6 g/L trisodium phosphate dodecahydrate, 40 g/L Difco Soytone (Thermo Fisher Scientific Inc.), 1 g/L ammonium chloride, 10 g/L glucose, 1 g/L magnesium sulfate heptahydrate, 0.01 g/L iron(II) sulfate heptahydrate, 0.005 g/L manganese(II) chloride tetrahydrate, 200 µL/L Adekanol (ADEKA Corporation), and 50 mg/L kanamycin sulfate). As a culture device, BMS-03PI manufactured by ABLE Corporation was used, and the agitation speed was set to 400 to 700 rpm, the air aeration rate was set to 1.5 L/minute, the culture temperature was set to 30°C, and the pH was set to 6.8 to 7.2. Note that the fluctuation of pH during the culture was controlled within the above range by adding 14% (w/v) ammonia water or 50% (w/v) phosphoric acid.
(3) A feeding pump was started when a DO (dissolved oxygen concentration) measured by a DO electrode attached to BMS-03PI exceeded 40% saturation. An operation of supplying a feeding medium (425 g/L D(+)-glucose, 124 g/L Yeast Extract Red Label (Oriental Yeast Co., Ltd.), 12.5 g/L magnesium sulfate heptahydrate, and 50 mg/L kanamycin sulfate) until the DO became 40% saturation or less again was continued until the completion of the culture.
(4) At a time point from 19 hours to 21 hours after the start of the culture, the culture temperature was changed to 25°C, the agitation speed was changed to 600 rpm, and IPTG was added at a final concentration of 0.1 mmol/L to induce expression of the Fc-binding protein.
(5) 48 hours after the start of the culture, the culture was terminated, and cultured bacterial cells were collected by centrifuging the culture fluid.
(6) Extraction was performed on the collected bacterial cells at room temperature for 2 hours, using 50 mmol/L Bis-Tris propane buffer (pH 10.0) containing 150 mmol/L sodium chloride, 2.4 mmol/L magnesium sulfate, 5000 Unit/L Benzonase (Merck KGaA), 0.006% (w/v) lysozyme, and 0.6% (w/v) Triton X-100 (trade name). Thereafter, the bacterial cell extract was centrifuged at 4°C at 15000 rpm for 20 minutes to obtain an extract containing the Fc-binding protein.
(7) The extract containing the Fc-binding protein, obtained in (6), was applied to an Econo-Pac column (Bio-Rad Laboratories, Inc.) packed with 5 mL of Ni-NTA agarose (Fujifilm Wako Pure Chemical Corporation) equilibrated in advance with 50 mmol/L Bis-Tris propane buffer (pH 10.0) containing 150 mmol/L sodium chloride. Washing was performed with 50 mmol/L Bis-Tris propane buffer (pH 10.0) containing 20 mmol/L imidazole and 150 mmol/L sodium chloride. Thereafter, elution was performed with 50 mmol/L Bis-Tris propane buffer (pH 10.0) containing 300 mmol/L imidazole and 150 mmol/L sodium chloride to obtain a crudely purified protein solution containing the Fc-binding protein.
(8) The crudely purified protein solution containing the Fc-binding protein, obtained in (7), was adjusted to pH 6.5 using 1 mol/L hydrochloric acid (Fujifilm Wako Pure Chemical Corporation).
(9) The crudely purified protein solution containing the Fc-binding protein, obtained in (8) and pH-adjusted, was applied to a Poly-Prep column (Bio-Rad Laboratories, Inc.) packed with 2 mL of IgG-Sepharose (Cytiva) equilibrated in advance with 50 mmol/L Bis-Tris propane buffer (pH 6.5) containing 150 mmol/L sodium chloride. After washing with the buffer used for equilibration, a purified Fc-binding protein was obtained by elution with 50 mmol/L Bis-Tris propane buffer (pH 8.5) containing 150 mmol/L sodium chloride. Note that the purified Fc-binding protein obtained using the plasmid capable of expressing OmpA-FcRn(B2M-GS-EC_m7b)-6HC (SEQ ID NO: 92) is also referred to as FcRnm7b-2 (corresponding to isoleucine (I) at position 22 to glycine (G) at position 432 of SEQ ID NO: 92). The purified Fc-binding protein obtained using the plasmid capable of expressing OmpA-FcRn(B2M-GS-EC_m10)-6HC (SEQ ID NO: 93) is also referred to as FcRnm10 (corresponding to isoleucine (I) at position 22 to glycine (G) at position 432 of SEQ ID NO: 93).
(10) The absorbance at a wavelength of 280 nm was measured for the purified protein solution obtained in (9) to calculate the purification yield.

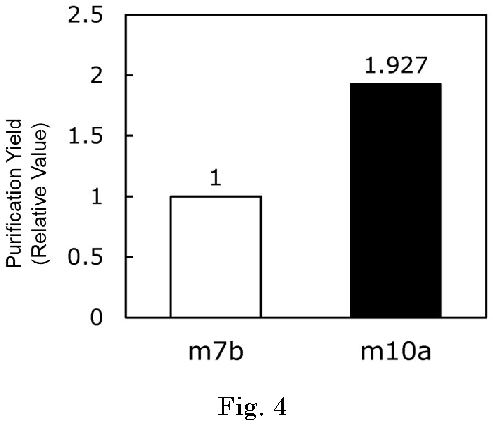

The results are shown in FIG. 4. Note that in FIG. 4, the purification yield is expressed as a relative value,with the purification yield of FcRnm7b-2 set to 1. It can be seen that the purification yield is further enhanced by introducing at least any one or more amino acid substitutions of Vα80D, Kα96E, and Nα172D.

### Example 10: Measurement of binding activity of Fc-binding protein to IgG

Measurement of the binding activity of the Fc-binding protein to IgG was performed by the below-described method.
(1) The purified Fc-binding protein FcRnm10 solution obtained in Example 9 was diluted with 50 mmol/L MES buffer (pH 6.5) containing 150 mmol/L sodium chloride to prepare solutions having Fc-binding protein concentrations of 500 µg/mL, 250 µg/mL, 125 µg/mL, 62.5 µg/mL, 31.3 µg/mL, and 15.6 µg/mL.
(2) A gamma globulin preparation (KM Biologics Co., Ltd.), which is a human antibody, was immobilized on wells of a 96-well microplate at 10 µg/well (at 4°C for 18 hours).
(3) Washing was performed with a washing buffer (50 mmol/L MES buffer (pH 6.5) containing 150 mmol/L sodium chloride), and blocking was performed with a buffer (pH 7.4) containing 2% (w/v) SKIM MILK (Becton, Dickinson and Company), 137 mmol/L sodium chloride, 8.1 mmol/L disodium hydrogen phosphate, 2.7 mmol/L potassium chloride, and 1.5 mmol/L potassium dihydrogen phosphate.
(4) After washing with the washing buffer, a solution containing the Fc-binding protein for evaluation of antibody-binding activity was added, and the Fc-binding protein and the immobilized gamma globulin were allowed to react with each other (at 30°C for 1 hour).
(5) After washing with the washing buffer, an Anti-FcRn monoclonal antibody (Mouse) (Santa Cruz Biotechnology, Inc.; sc-271745) diluted 10000-fold with the washing buffer was added at 100 µL/well, and allowed to react (at 30°C for 1 hour).
(6) After washing with the washing buffer, Anti Mouse IgG-heavy and light chain, HRP (Goat) (Bethyl Laboratories: A90-216P), diluted 10000-fold with the washing buffer was added at 100 µL/well, and allowed to react (at 30°C for 1 hour).
(7) After washing with the washing buffer, TMB Peroxidase Substrate (KPL) was added at 50 µL/well. Color development was stopped by adding 1 M phosphoric acid at 50 µL/well, and absorbance at 450 nm was measured with a microplate reader (Tecan Group Ltd.).

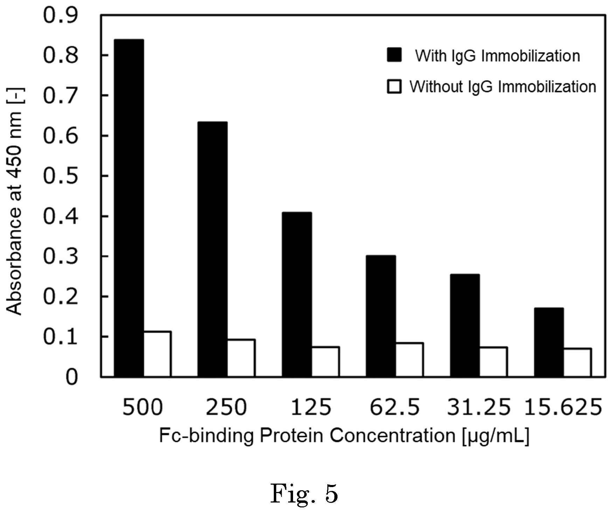

The results are shown in FIG. 5. Note that "No IgG immobilization" in FIG. 5 is control data obtained by adding the buffer (pH 7.4) containing 137 mmol/L sodium chloride, 8.1 mmol/L disodium hydrogen phosphate, 2.7 mmol/L potassium chloride, and 1.5 mmol/L potassium dihydrogen phosphate, instead of the gamma globulin preparation in (2). The absorbance is higher in the wells with IgG immobilization than in the wells without IgG immobilization, and the absorbance depends on the concentration of the Fc-binding protein. Accordingly, it can be seen that the Fc-binding protein of the present disclosure has binding activity for IgG.

### Example 11: Immobilization of Fc-binding protein to insoluble carrier

Two grams of a porous hydrophilic polymer (Tosoh Corporation, G5000PW) for adsorbents was taken as a suction-dried gel. After activating the hydroxy groups on the surface of the gel with iodoacetyl groups, 10 mg of FcRnm10 obtained in Example 9 was allowed to react with the gel. As a result, an OmpA-FcRn(B2M-GS-EC_m10)-6HC-immobilized gel was obtained.

### Example 12: Antibody purification using antibody adsorbent-packed column (Part 1)

(1) An antibody adsorbent column was prepared by packing 0.83 mL of the OmpA-FcRn(B2M-GS-EC_m10)-6HC-immobilized gel obtained in Example 11 (hereinafter also simply referred to as "antibody adsorbent") into a stainless steel column (Tosoh Corporation) having a diameter of 4.6 mm and a length of 50 mm.
(2) After connecting the antibody adsorbent column prepared in (1) to a Nexera system (Shimadzu Corporation), the column was equilibrated by feeding 5 column volumes (hereinafter referred to as CV; 1 CV = 0.83 mL) of 50 mmol/L MES buffer (pH 6.5) containing 150 mmol/L sodium chloride (hereinafter also referred to as "equilibration liquid") to the column at a flow rate of 0.4 mL/min.
(3) After diluting a monoclonal antibody (Rituxan, Zenyaku Kogyo Co., Ltd.) to 1.0 mg/mL with the equilibration solution used in (2), 30 µL of the diluted liquid (30 µg load as an antibody) was fed to the antibody adsorbent column at a flow rate of 0.4 mL/min, whereby the antibody was adsorbed to the antibody adsorbent.
(4) Subsequently, after feeding the equilibration liquid to the antibody adsorbent column at a flow rate of 0.4 mL/min for 10 minutes, an eluent (50 mmol/L Tris buffer (pH 8.5) containing 150 mmol/L sodium chloride) was fed to the antibody adsorbent column with a linear gradient such that the eluent became 100% in 20 minutes, whereby the antibody adsorbed to the antibody adsorbent was eluted. After the antibody elution, the eluent was subsequently fed to the antibody adsorbent column at a flow rate of 0.4 mL/min for 10 minutes, and then the buffer used for equilibration was flowed through the antibody adsorbent column for 10 minutes to perform equilibration.

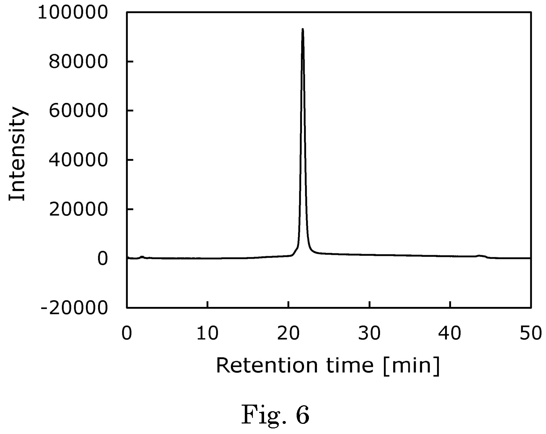

A chromatogram obtained by separating the monoclonal antibody is shown in FIG. 6. In FIG. 6, the monoclonal antibody is loaded onto the antibody adsorbent column at a time point of 0 minutes. The monoclonal antibody was eluted between 10 minutes and 30 minutes, during which the linear gradient was applied, and it has been confirmed that the antibody can be separated using the column packed with the gel on which the Fc-binding protein of the present disclosure is immobilized.

### Example 13: Antibody purification using antibody adsorbent column (Part 2)

Separation tests were performed not only for the monoclonal antibody described in Example 12 (3) but also for substances having other Fc regions. In the tests, as the substances having an Fc region, an antibody-drug conjugate (ADC) and an Fc fusion protein were used in addition to the monoclonal antibody.
(1) An antibody adsorbent column was prepared by the method described in Example 12 (1), and the column was equilibrated by the method described in Example 12 (2).
(2) Adsorption to the antibody adsorbent was performed in the same manner as in Example 12 (3), except that any one of the substances shown in Table 6 was used as the substance having an Fc region. The antibody adsorbed to the adsorbent was eluted by the method described in Example 12 (4).

### [Table 6]

**Table 6**

| | Substance having an Fc region | | | |
|---|---|---|---|---|
| | Trade name | Manufacturer | Aspect | Sequence of Fc region |
| <1> | Synagis | Astra Zeneca | Monoclonal antibody | SEQ ID NO: 102 |
| <2> | Avastin | CHUGAI PHARMACEUTICAL | Monoclonal antibody | SEQ ID NO: 103 |
| <3> | Erbitux | Merck Biopharma | Monoclonal antibody | SEQ ID NO: 104 |
| <4> | Herceptin | CHUGAI PHARMACEUTICAL | Monoclonal antibody | SEQ ID NO: 105 |
| <5> | Kadcyla | CHUGAI PHARMACEUTICAL | Antibody-drug conjugate (ADC) | SEQ ID NO: 106 |
| <6> | Remicade | Mitsubishi Tanabe Pharma | Monoclonal antibody | SEQ ID NO: 107 |
| <7> | Humira | Eisai | Monoclonal antibody | SEQ ID NO: 108 |
| <8> | Actemra | CHUGAI PHARMACEUTICAL | Monoclonal antibody | SEQ ID NO: 109 |
| <9> | Vectibix | Takeda Pharmaceutical | Monoclonal antibody | SEQ ID NO: 110 |
| <10> | Ranmark | Daiichi Sankyo | Monoclonal antibody | SEQ ID NO: 111 |
| <11> | Emgality | Daiichi Sankyo | Monoclonal antibody | SEQ ID NO: 112 |
| <12> | Zaltrap | Sanofi | Fc fusion protein | SEQ ID NO: 113 |
| <13> | Trulicity | Sumitomo Pharma | Fc fusion protein | SEQ ID NO: 114 |
| <14> | Orencia | ONO PHARMACEUTICAL | Fc fusion protein | SEQ ID NO: 115 |
| <15> | Romiplate | Kyowa Kirin | Fc fusion protein | SEQ ID NO: 116 |

Note that the amino acid sequence of the Fc region possessed by the monoclonal antibody described in Example 12 (3), that is, Rituxan, is shown in SEQ ID NO: 101. Additionally, among the substances having an Fc region that are shown in Table 6, the amino acid sequence of the Fc region possessed by Synagis is shown in SEQ ID NO: 102, the amino acid sequence of the Fc region possessed by Avastin is shown in SEQ ID NO: 103, the amino acid sequence of the Fc region possessed by Erbitux is shown in SEQ ID NO: 104, the amino acid sequence of the Fc region possessed by Herceptin is shown in SEQ ID NO: 105, the amino acid sequence of the Fc region possessed by Kadcyla is shown in SEQ ID NO: 106, the amino acid sequence of the Fc region possessed by Remicade is shown in SEQ ID NO: 107, the amino acid sequence of the Fc region possessed by Humira is shown in SEQ ID NO: 108, the amino acid sequence of the Fc region possessed by Actemra is shown in SEQ ID NO: 109, the amino acid sequence of the Fc region possessed by Vectibix is shown in SEQ ID NO: 110, the amino acid sequence of the Fc region possessed by Ranmark is shown in SEQ ID NO: 111, the amino acid sequence of the Fc region possessed by Emgality is shown in SEQ ID NO: 112, the amino acid sequence of the Fc region possessed by Zaltrap is shown in SEQ ID NO: 113, the amino acid sequence of the Fc region possessed by Trulicity is shown in SEQ ID NO: 114, the amino acid sequence of the Fc region possessed by Orencia is shown in SEQ ID NO: 115, and the amino acid sequence of the Fc region possessed by Romiplate is shown in SEQ ID NO: 116.

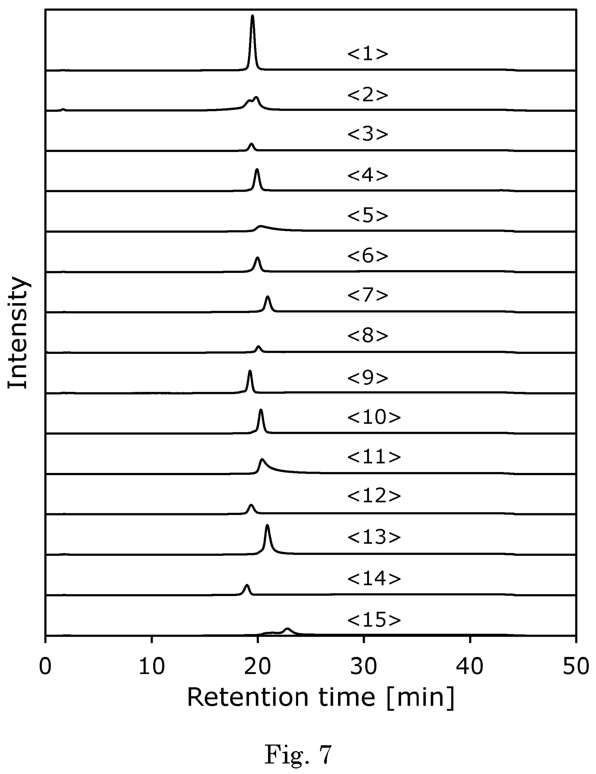

A chromatogram obtained by separating the substances having an Fc region that are shown in Table 6 is shown in FIG. 7. Similarly to FIG. 6, each antibody was loaded onto the antibody adsorbent column at the time point of 0 minutes. Each protein was eluted between 10 minutes and 30 minutes, while the linear gradient was being applied. It has been confirmed that, even for substances other than Rituxan (Example 12), a substance having an Fc region can be separated using the column packed with the gel on which the Fc-binding protein of the present disclosure is immobilized. Regardless of the form, such as a monoclonal antibody, an ADC, or an Fc fusion protein, any substance with an Fc region can be separated.

### Example 14: Evaluation of alkali resistance of Fc-binding protein

### (1) Preparation of protein

(1-1) An *Escherichia coli* BL21 (DE3) strain transformed with an expression vector pET-eFcRn was prepared by the method disclosed in Example 1 of JP2018-183087A.
(1-2) A purified Fc-binding protein, FcRnWT (corresponding to isoleucine (I) at position 34 to histidine (H) at position 437 of SEQ ID NO: 118), was obtained by performing the same operation as in Example 9, except that the *Escherichia coli* strain prepared in (1-1) was used.

Note that the amino acid sequence of the polypeptide expressed by the expression vector pET-eFcRn is shown in SEQ ID NO: 118, and the sequence of the polynucleotide encoding the polypeptide is shown in SEQ ID NO: 119. In SEQ ID NO: 118, the sequence from methionine (Met) at position 1 to alanine (Ala) at position 26 is a MalE signal peptide, the sequence from lysine (Lys) at position 27 to glycine (Gly) at position 33 is a linker sequence, the sequence from isoleucine (Ile) at position 34 to methionine (Met) at position 132 is a β2-microglobulin region of human FcRn β-chain (region from position 21 to position 119 of SEQ ID NO: 2), the sequence from glycine (Gly) at position 133 to serine (Ser) at position 157 is a GS linker sequence, the sequence from alanine (Ala) at position 158 to serine (Ser) at position 431 is an extracellular region of human FcRn α-chain (region from position 24 to position 297 of SEQ ID NO: 1), and the sequence from histidine (His) at position 432 to position 437 is a tag sequence.

### (2) Evaluation of alkali resistance

(2-1) FcRnWT obtained in (1), FcRnm7a and FcRnm7b obtained in Example 7, and FcRnm10 obtained in Example 9 were each adjusted to a final concentration of 0.1 mg/mL.
(2-2) NaOH was added to the Fc-binding protein solution prepared in (2-1) to a final concentration of 0.04 mol/L or 0.08 mol/L. The resultant mixture was left to stand at room temperature for 1.5 hours.
(2-3) To the solution after standing in (2-2), 50 mmol/L MES buffer (pH 5.5) containing 150 mmol/L sodium chloride was added to adjust the pH to 6.0.
(2-4) The protein solution containing the Fc-binding protein obtained in (2-3) and pH-adjusted, was applied to a Poly-Prep column (Bio-Rad Laboratories, Inc.) packed with 0.2 mL of IgG Sepharose (Cytiva) equilibrated in advance with 50 mmol/L Bis-Tris propane buffer (pH 6.0) containing 150 mmol/L sodium chloride. After washing with the equilibration buffer, the Fc-binding protein was obtained by elution with 50 mmol/L Bis-Tris propane buffer (pH 8.5) containing 150 mmol/L sodium chloride. Note that the same operation was also performed on the Fc-binding protein solution prepared in (2-1).
(2-5) The eluted fraction (purified solution) obtained in (2-4) was subjected to SDS-PAGE (sodium dodecyl sulfate-polyacrylamide gel electrophoresis) and stained with a CBB (Coomassie Brilliant Blue) staining solution (Fujifilm Wako Pure Chemical Corporation). Then, the band intensity corresponding to the Fc-binding protein was quantified using image analysis software (ImageQuant TL 10.0, Cytiva).

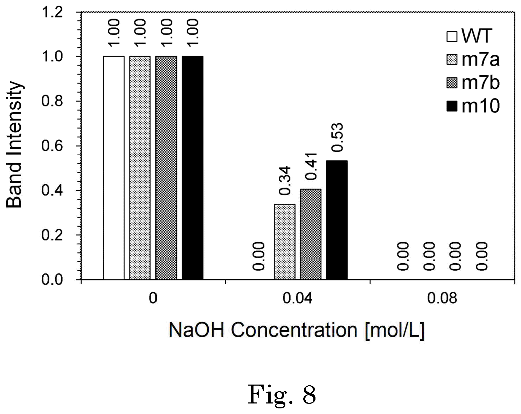

The results are shown in FIG. 8. Note that in FIG. 8, the band intensity is expressed as a relative value, with the value of the eluted fraction of each Fc-binding protein to which NaOH was not added set to 1. It was found that the binding activity for IgG was lost in FcRnWT treated with 0.04 mol/L NaOH, whereas the binding activity for IgG was retained in FcRnm7a, FcRnm7b, and FcRnm10. Additionally, the binding activity for IgG was retained to a greater extent in FcRnm7b and FcRnm10 than in FcRnm7a. That is, it has been revealed that FcRnm7b and FcRnm10 possess greater alkali resistance than FcRnm7a. Furthermore, it has been revealed that FcRnm10 possesses greater alkali resistance than FcRnm7b.

That is, it has been shown that the Fc-binding protein in which cysteine at position 274 of SEQ ID NO: 1 is substituted with another amino acid possesses not only enhanced productivity but also enhanced alkali resistance.

### Industrial Applicability

An Fc-binding protein of the present disclosure is a protein obtained by substituting an amino acid residue at a specific position in the extracellular region of the human neonatal Fc receptor α-chain with another amino acid residue. The Fc-binding protein has significantly enhanced productivity compared to conventional Fc-binding proteins. The Fc-binding protein is useful as a ligand for an adsorbent for separating a substance having an Fc region, such as an immunoglobulin, and is suitable for industrial production due to the enhancement in productivity. Additionally, in one aspect, an Fc-binding protein of the present disclosure possesses significantly enhanced alkali resistance compared to conventional Fc-binding proteins. By using the Fc-binding protein as a ligand for an antibody adsorbent, an adsorbent having high-alkali-washing resistance can be obtained. Accordingly, the Fc-binding protein can contribute to cost reduction in the industrial production of antibody drugs.

## Claims

1. An Fc-binding protein selected from any one of the following <a> to <c>:
<a> an Fc-binding protein comprising amino acid residues from alanine at position 24 to serine at position 297 of the amino acid sequence of SEQ ID NO: 1 and amino acid residues from isoleucine at position 21 to methionine at position 119 of the amino acid sequence of SEQ ID NO: 2, with the proviso that at least the amino acid substitutions of the following (1) to (7) are introduced into the amino acid residues:
(1) substitution of cysteine at position 71 of SEQ ID NO: 1 with arginine;
(2) substitution of asparagine at position 78 of SEQ ID NO: 1 with aspartic acid;
(3) substitution of arginine at position 192 of SEQ ID NO: 1 with leucine;
(4) substitution of asparagine at position 196 of SEQ ID NO: 1 with aspartic acid;
(5) substitution of glutamine at position 232 of SEQ ID NO: 1 with leucine;
(6) substitution of lysine at position 295 of SEQ ID NO: 1 with glutamic acid; and
(7) substitution of cysteine at position 274 of SEQ ID NO: 1 with another amino acid;
<b> an Fc-binding protein comprising amino acid residues from alanine at position 24 to serine at position 297 of the amino acid sequence of SEQ ID NO: 1 and amino acid residues from isoleucine at position 21 to methionine at position 119 of the amino acid sequence of SEQ ID NO: 2, with the proviso that, into the amino acid residues, the amino acid substitutions of (1) to (7) above are introduced, and furthermore, any one or more of substitutions, deletions, insertions, and additions of one or several amino acid residues at one or several positions other than the amino acid substitutions of (1) to (7) above are introduced, and wherein the Fc-binding protein has antibody-binding activity; and
<c> an Fc-binding protein comprising an amino acid sequence that has an identity of 70% or more to the entire amino acid sequence composed of the amino acid sequence from alanine at position 24 to serine at position 297 of the amino acid sequence of SEQ ID NO: 1 and the amino acid sequence from isoleucine at position 21 to methionine at position 119 of the amino acid sequence of SEQ ID NO: 2, with the proviso that the amino acid substitutions of (1) to (7) above are introduced into the entire amino acid sequence, wherein the amino acid sequence of the Fc-binding protein has the amino acid substitutions of (1) to (7) above maintained therein, and wherein the Fc-binding protein has antibody-binding activity.

2. The Fc-binding protein according to claim 1, selected from any one of the following <d> to <f>:
<d> an Fc-binding protein comprising amino acid residues from alanine at position 24 to serine at position 297 of the amino acid sequence of SEQ ID NO: 1 and amino acid residues from isoleucine at position 21 to methionine at position 119 of the amino acid sequence of SEQ ID NO: 2, with the proviso that the amino acid substitutions of (1) to (7) above and any one or more of the amino acid substitutions of the following (8) to (10) are introduced into the amino acid residues:
(8) substitution of valine at position 80 of SEQ ID NO: 1 with aspartic acid;
(9) substitution of lysine at position 96 of SEQ ID NO: 1 with glutamic acid; and
(10) substitution of asparagine at position 172 of SEQ ID NO: 1 with aspartic acid;
<e> an Fc-binding protein comprising amino acid residues from alanine at position 24 to serine at position 297 of the amino acid sequence of SEQ ID NO: 1 and amino acid residues from isoleucine at position 21 to methionine at position 119 of the amino acid sequence of SEQ ID NO: 2, with the proviso that, into the amino acid residues, the amino acid substitutions of (1) to (7) above and at least any one of the amino acid substitutions of (8) to (10) above are introduced, and furthermore, any one or more of substitutions, deletions, insertions, and additions of one or several amino acid residues at one or several positions other than the amino acid substitutions of (1) to (10) above are introduced, and wherein the Fc-binding protein has antibody-binding activity; and
<f> an Fc-binding protein comprising an amino acid sequence that has an identity of 70% or more to the entire amino acid sequence composed of the amino acid sequence from alanine at position 24 to serine at position 297 of the amino acid sequence of SEQ ID NO: 1 and the amino acid sequence from isoleucine at position 21 to methionine at position 119 of the amino acid sequence of SEQ ID NO: 2, with the proviso that the amino acid substitutions of (1) to (7) above and at least any one of the amino acid substitutions of (8) to (10) above are introduced into the entire amino acid sequence, wherein the amino acid sequence of the Fc-binding protein has the amino acid substitutions of (1) to (10) above maintained therein, and wherein the Fc-binding protein has antibody-binding activity.

3. The Fc-binding protein according to claim 2, selected from any one of the following <g> to <i>:
<g> an Fc-binding protein comprising amino acid residues from alanine at position 24 to serine at position 297 of the amino acid sequence of SEQ ID NO: 1 and amino acid residues from isoleucine at position 21 to methionine at position 119 of the amino acid sequence of SEQ ID NO: 2, with the proviso that at least the amino acid substitutions of (1) to (10) above are introduced into the amino acid residues;
<h> an Fc-binding protein comprising amino acid residues from alanine at position 24 to serine at position 297 of the amino acid sequence of SEQ ID NO: 1 and amino acid residues from isoleucine at position 21 to methionine at position 119 of the amino acid sequence of SEQ ID NO: 2, with the proviso that, into the amino acid residues, the amino acid substitutions of (1) to (10) above are introduced, and furthermore, any one or more of substitutions, deletions, insertions, and additions of one or several amino acid residues at one or several positions other than the amino acid substitutions of (1) to (10) above are introduced, and wherein the Fc-binding protein has antibody-binding activity; and
<i> an Fc-binding protein comprising an amino acid sequence that has an identity of 70% or more to the entire amino acid sequence composed of the amino acid sequence from alanine at position 24 to serine at position 297 of the amino acid sequence of SEQ ID NO: 1 and the amino acid sequence from isoleucine at position 21 to methionine at position 119 of the amino acid sequence of SEQ ID NO: 2, with the proviso that the amino acid substitutions of (1) to (10) above are introduced into the entire amino acid sequence, wherein the amino acid sequence of the Fc-binding protein has the amino acid substitutions of (1) to (10) above maintained therein, and wherein the Fc-binding protein has antibody-binding activity.

4. The Fc-binding protein according to claim 1, wherein the other amino acid in (7) above is any one of serine, glutamic acid, and histidine.

5. A polynucleotide encoding the Fc-binding protein according to any one of claims 1 to 4.

6. An expression vector comprising the polynucleotide according to claim 5.

7. A transformant capable of producing an Fc-binding protein, obtained by transforming a host with the expression vector according to claim 6.

8. The transformant according to claim 7, wherein the host is *Escherichia coli*.

9. A method of producing an Fc-binding protein, comprising the steps of: culturing the transformant according to claim 7 to produce the Fc-binding protein; and collecting the produced Fc-binding protein from the resultant culture product.

10. An antibody adsorbent obtained by immobilizing the Fc-binding protein according to any one of claims 1 to 4 on an insoluble carrier.

11. A method of separating a substance having an Fc region, comprising the steps of: adding a solution containing a substance having an Fc region to a column packed with the adsorbent according to claim 10 to adsorb the substance having an Fc region to the adsorbent; and eluting the substance having an Fc region adsorbed to the adsorbent, using an eluent.
